# EUROPEAN PATENT SPECIFICATION

(11) **EP 0 652 769 B1**
(45) Date of publication and mention of the grant of the patent: **06.03.1996**
(21) Application number: 93916110.5
(22) Date of filing: 20.07.1993
(51) Int. Cl.: A61K 39/00, C07K 14/435, C12N 9/64, C12N 15/12

(54) **VACCINES AGAINST METAZOAN PARASITES**
Impfstoffe gegen Metazoen-Parasiten
VACCINS CONTRE LES PARASITES METAZOAIRES

(30) Priority: 21.07.1992 GB 9215505; 19.08.1992 GB 9217635
(43) Date of publication of application: 17.05.1995
(73) Proprietor: MALLINCKRODT VETERINARY, INC., Mundelein, Illinois 60060 (US)
(72) Inventor: SMITH, William, David, Edinburgh EH9 1AZ (GB); SMITH, Stuart, Kevin, Edinburgh EH16 6UZ (GB); MURRAY, Jacqueline, Apt. H203 Newport Crossing, Bellevue, WA 98056 (US); LIDDELL, Susan, Edinburgh EH1 1SJ (GB); KNOX, David Patrick, Cockenzie, East Lothian EH32 0JD (GB)
(74) Representative: Dzieglewska, Hanna Eva
(86) International application number: GB9301521
(87) International publication number: WO9402169

(56) References cited:
- WO-A-88/00835
- WO-A-90/11086
- INTERNATIONAL JOURNAL FOR PARASITOLOGY vol. 23, no. 2 , April 1993 pages 271 - 280 TREVOR S. SMITH ET AL. 'PURIFICATION AND EVALUATION OF THE INTEGRAL MEMBRANE PROTEIN H11 AS A PROTECTIVE ANTIGEN AGAINST HAEMONCHUS CONTORTUS'

## Description

This invention relates to novel metazoan parasite antigens and their use in the control of disease caused by helminth and other metazoan parasites, particularly helminth and arthropod parasites in domestic animals.

Each species of domestic animal can be parasitised by a number of different parasites, including many species of helminths and arthropods, a process which usually causes disease. Some of these diseases are of considerable economic importance: an example is haemonchosis which is caused by the helminth Haemonchus contortus, a nematode which parasitises the abomasum or true stomach of various ruminants including sheep. Juvenile and adult Haemonchus are blood suckers which attach by their mouthparts to small blood vessels within the gastric mucosa. Heavily parasitised sheep lose large amounts of blood leading to anaemia, ill-thrift and frequently death. Haemonchosis is more prevalent in tropical and subtropical parts of the world because the non-parasitic stages of H. contortus on the pasture are susceptible to low temperatures and often do not survive winter in temperate climates.

Also of very considerable significance from an economic point of view are the non-blood feeding nematodes Ostertagia ostertagi and Ostertagia (Teladorsagia) circumcincta (O.circumcincta has recently been reclassified as T.circumcincta, although the new name is not yet in wide usage). Cattle and sheep infected with O.ostertagi and O.circumcincta respectively, fail to thrive and may die if untreated and Ostertagiosis represents one of the major helminth infections causing problems in animal husbandry today.

Other parasitic worms of economic importance include the various species of the following helminth families:-Trichostrongylus, Nematodirus, Dictyocaulus, Cooperia, Ascaris, Dirofilaria, Trichuris, Strongylus, Fasciola, Oesophagostomum, Bunostomum and Metastrongylus. In addition to domestic livestock, pets and humans may also be infected, not infrequently with fatal results and helminth infections and infestations thus pose a problem of considerable worldwide significance.

Other metazoan parasites of significance include many arthropod species especially members of the arachnid and insect classes and in particular ectoparasites such as the blood-feeding (haematophagous) insects (eg. members of the insect divisions exopterygota and endopterygota), blowfly (Lucilia) and ticks.

Particular mention may be made in this regard of ticks of the species Boophilus spp, Amblyomma spp, Argas spp, Rhipicephalus spp, Hyalomma spp, Ornithodorus spp, Dermacentor spp, Ixodes spp; flies, particularly Lucilia spp and myiasis, sucking and biting flies, such as Stomoxys calcitrans, Oestrus ovis, Hydrotaea irritans, Gasterophilus spp, Simulium spp, Lyperosia irritans, Haematobia spp, Tabanus spp, Chrysomyia spp, Calliphora spp, Phlebotomus spp, Glossina spp, Hypoderma spp, Dermatobia spp, and Cochliomyia spp, lice eg. Haematopinus eurysternus, Linognathus vittuli, Solenopotes canillatus, Linognathus ovillus, and Menacanthus spp; mites such as Psoroptes spp, Demodex spp, Sarcoptes spp, Chorioptes spp, Psorergates spp, Dermanyssus spp, Ornithonyssus spp, Otodectes spp and Notoedres spp; fleas eg. Ctenocenhalides canis and C. felis; keds eg. Melophagus ovinus and bugs such as Cimex spp.

In addition to their own parasitic effects, many blood-feeding arthropods are important in the transmission of a variety of pathogens, eg. pathogenic protozoa or viruses, of medical and veterinary significance. Livestock producers annually experience substantial losses due to haematophagus arthropods and the diseases they transmit.

Control of helminth and arthropod parasites of grazing livestock currently relies primarily on the use of anthelmintics, endectosides and/or insecticides combined with pasture management. Such techniques are often unsatisfactory firstly, because the drugs have to be administered frequently, secondly because resistance against anthelmintic and arthropodicidal drugs is becoming increasingly widespread and thirdly because appropriate pasture management is often not possible on some farms and even where it is, it can place constraints on the best use of available grazing.

Attempts have been made to control parasites of domestic animals by immunological means. With very few exceptions (e.g. the cattle lungworm, Dictvocaulus viviparus) this has not proved possible and no commercially available vaccines exist to date for gastrointestinal nematodes of ruminants including Haemonchus and Ostertagia, and the major arthropod parasites.

Over the last few years however, several reports have described novel proteins which have been isolated from Haemonchus and which have potential as protective antigens not only against Haemonchus but also against a range of related endoparasites of domestic animals (reviewed by Emery and Wagland 1991 Parasitology Today 7 347-349).

Contortin, a helical polymeric extracelluar protein which is associated with the luminal surface of Haemonchus contortus intestinal cells, was first described by Munn in 1977 (Tissue and Cell 1977, 9 23-34) and when administered as a crude extract in large doses (several milligrams), was shown subsequently to protect lambs against haemonchosis (Munn 1987, Parasitology 94 385-397). Because of the large doses required and the impurity of preparations injected, it was not clear whether the protection afforded in these experiments was in fact due to contortin per se or to other antigens which were contaminating the vaccine. Indeed further work showed that sheep immunised with such preparations of contortin also had antibodies to a gut membrane protein doublet termed H110D. It was later shown that sheep immunised with small doses (a few hundred micrograms) of highly purified H110D were substantially protected against haemonchosis (Munn, WO88/00835; Munn and Smith WO90/11086). H110D now represents the most promising vaccine candidate against Haemonchus to date. A third Haemonchus gut membrane protein with protective antigen properties has also been discovered and termed H45 (Munn and Smith WO90/11086).

The literature also contains reports of less successful attempts to identify protective antigens in Haemonchus and other helminth species. Thus for example a 45kd membrane glycoprotein has been isolated from adult Haemonchus which conferred about 50% protection in terms of worm numbers when used as an immunogen in guinea pigs challenged with this parasite (Sharp, Wagland and Cobon WO92/13889). Its effect in sheep was not described. There is no indication of the native location of this antigen in the worm, and certainly no indication that it is a luminal gut surface protein. Much cruder fractions of Haemonchus membrane extracts, which almost certainly contained H110D, were claimed to reduce ovine egg and worm counts by up to 88 and 75% respectively despite no information being provided concerning either group size or variation between sheep. Haemonchus contortus does not naturally parasitise guinea pigs. Instead of persisting for several weeks as it does in a natural host such as the sheep, Haemonchus infection of guinea pigs is severely truncated so that nearly all worms are rejected between 5 and 7 days after infection well before they reach the adult stage (Wagland, Abeydeera, Rothwell and Ouwerkerk 1989 International Journal for Parasitology 19 301-305). In the guinea pig challenge model used in WO92/13889 the animals were killed 5 or 6 days after challenge so that any difference between vaccinates and controls merely reflected an acceleration of the rejection phenomenon by 1 or 2 days. This very slight difference contrasts with the effects described in sheep in the present invention (see also WO88/00835 and W090/11086 mentioned above) and casts doubt on the value of this laboratory animal model in this context. This doubt is emphasised later in the present specification by experiments where it was found that a wheatgerm lectin binding fraction of a Haemonchus membrane extract was highly protective for sheep, yet in W092/13889 an essentially similar preparation was reported not to work in the guinea pig model.

WO89/00163 describes the cloning and expression of a gene coding for a 41Kd protein from Trichostrongylus colubriformis, a member of the same taxonomic family as Haemonchus and a parasite of the small intestine of the sheep. The protein has not been characterised and whilst vaccination of sheep with proteins from recombinant organisms expressing the gene was alleged to result in reduced egg and worm counts after challenge with Haemonchus as compared with control sheep, this reduction was not statistically significant and, unless it can be improved upon, the degree of protection afforded will not be commercially useful.

A water soluble cysteine protease has recently been isolated from Haemonchus contortus and the gene encoding it has been cloned and expressed (Cox, Pratt, Hageman and Boisvenue 1990 Molecular and Biochemical Parasitology 41 25-34). This protease has a molecular weight of 35Kd as judged by SDS-PAGE run under reducing conditions, although a more heavily glycosylated version with a molecular weight of 37Kd was also identified. It was hypothesised that this protease, which putatively degrades fibrinogen, prevents host blood from clotting when Haemonchus is feeding, presumably enhancing both ingestion and digestion of the blood meal. It was claimed that sheep repeatedly injected with a few hundred micrograms of a fraction enriched for the native cysteine protease were protected against Haemonchus because they had reduced faecal egg counts and smaller worm burden than control sheep after challenge with 2,500 Haemonchus larvae (Boisvenue, Stiff, Tonkinson, Cox and Hageman 1990 Proceedings of the VIIth International Congress of Parasitology p.475). However, the details of these experiments which are published in US Patents 408339 and 487181 reveal that the actual differences in worm and egg counts between vaccinate and control groups were marginal and not statistically significant.

A 44Kd protease has been purified from exsheathing fluid of Haemonchus third stage larvae (Gamble, Purcell and Fetterer 1989 Molecular and Biochemical Parasitology 33 49-58). This protease, which was defined as a metallo-protease and was not inhibited by pepstatin, mediates ecdysis and was not tested for protective function. Maki and Yanagisawawa (Journal of Helminthology 1986, 60, 31-37) described carboxyl (aspartyl) and thiol protease activities in adult Schistosoma mansoni, Dirofilaria immitis, Angiostrongylus cantonensis and Ascaris suum. However, these activities were not fractionated or characterised in terms of molecular weights and no attempts were made to determine whether they might be candidate protective antigens.

A 31 Kd glycoprotein has been isolated from third stage O. circumcincta larvae (McGillivery, Young, Riffkin and Adler 1989 International Journal for Parasitology 19 473-478). Sheep immunised with this were partially protected against a challenge infection (WO91/01150).

Two low molecular weight proteins of 11 and 17 Kd have been isolated from excretory-secretory products of T. colubriformis (Savin et al., 1990, Molecular and Biochemical Parasitology 41 167-176; Dolpheide et al., 1991, Molecular and Biochemical Parasitology 45 101-108). Low or variable degrees of protection have been reported when recombinant versions of these antigens have been used to immunise sheep against challenge with T. colubriformis (Emery and Wagland 1991).

As far as arthropod parasites are concerned, the literature contains fewer reports of proposed vaccines and these concentrate mainly on cattle ticks, particularly the tick Boophilus microplus. Thus, Johnson et al. in 1986 proposed that protective antigens against B. microplus could be found in both soluble and insoluble fractions of extracts derived from adult female ticks (Johnson et al., 1986, Int. J. Parasitol, 16(1): 27-34). Since then reports have appeared in the literature proposing immunisation of cattle against ticks using tick antigens derived from extracts of the gut of adult ticks, tick larval extracts and from salivary gland components (see for example Willadsen et al., 1989, Int. J. Parasitol. 18(2): 183-189; Wong et al., 1990, Parasite Immunology, 12: 75-83; Jackson et al., 1990, Parasite Immunology, 12: 141-151).

There is a continuing need for new and improved metazoan parasite vaccines and in particular for a vaccine which may be used across a broad range of helminth and/or arthropod species.

The present invention accordingly seeks to provide novel antigens for use as metazoan parasite vaccines and in particular as protective immunogens in the control of diseases caused by helminth and other metazoan parasites.

More specifically, the present invention is based on the finding that the gut of H. contortus contains a proteolytically active glycoprotein complex which we have termed H-gal-GP (Haemonchus galactose-containing glycoprotein) and which is capable of conferring immunity in animals against Haemonchus and related parasites. Glycoprotein complexes analogous to H-gal-GP, which we have termed Oo-gal-GP and Oc-gal-GP, have been isolated from extracts of Ostertagia ostertagi and Ostertagia circumcincta respectively, and similar proteolytic activity has been observed in other helminths eg. Trichostrongylus. Such complexes when liberated from the membranes in which they are integrated, for example by the use of detergents such as Triton® X-100, are novel and of use in the manufacture of vaccines against helminth infections. Enzymes present in helminths are also observed in other metazoan parasites such as blowfly larvae.

According to one aspect, the present invention thus provides a protective metazoan parasite antigen or antigenic fragments, components or precursors thereof and functionally-equivalent derivatives, analogues, or variants thereof having protective antigenic activity against one or more metazoan parasites, which antigen is characterised by:
(a) possessing proteolytic activity;
(b) in native form being an integral membrane protein or protein complex, present in the intestinal brush border of the parasite gut;
(c) being capable of binding to pepstatin; and
(d) being capable of binding to wheatgerm lectin and to lectins having specificity for β-linked N-acetylgalactosamine.

A further aspect of the invention provides such protective antigens and antigenic fragments, components or precursors and functionally-equivalent derivatives, analogues or variants thereof for use in stimulating immune responses against metazoan parasites in a human or non-human, preferably mammalian, especially preferably ruminant, animal.

A precursor for the antigen in question may be a larger protein which is processed, eg. by proteolysis, to yield the antigen per se. Such precursors may take the form of zymogens, ie. inactive precursors of enzymes, activated by proteolytic cleavage, for example analogous to the pepsin/pepsinogen system or the well known zymogens involved in the blood clotting cascade.

The novel antigens according to the invention are not recognised by sera from naturally immune animals. In other words they are not normally, in native form, accessible to the immune system of the infected host and are thus "hidden", "concealed" or "cryptic" antigens.

As regards the lectin-binding characteristics of the novel antigens of the invention, binding to Con A, pea and Lotus lectins has also been demonstrated, indicating the presence of mannose and/or fucose residues. No binding is observed to lectins such as Datura and Bandeiraea indicating a lack of N-acetylglucosamine containing structures, but as mentioned above, binding to lectins having specificity for N-acetylgalactosamine, in particular β-linked N-acetylgalactosamine, is pronounced, particularly to peanut and jacalin lectins.

As regards proteolytic activity, whilst this has been demonstrated using general protease substrates such as azocollagen, azocasein, haemoglobin and gelatin, more specific activities have also been observed, most notably aspartyl proteinase-like and neutral endopeptidase-like activity. Thus for example, aspartyl proteinase-like activity has been demonstrated in both H and O-gal-GP preparations and in helminths such as Trichostrongylus. Neutral endopeptidase-like activity is contained in extracts containing the antigens according to the invention. Moreover, insofar as the specific antigen H-Gal-GP is concerned, preliminary nucleotide sequence data from a CDNA clone encoding a component of H-gal-GP indicates substantial homology with known neutral endopeptidase enzymes.

Thus, viewed from a different aspect, the invention also provides a protective metazoan parasite antigen or antigenic fragments, components or precursors thereof and functionally-equivalent derivatives, analogues or variants thereof having protective antigenic activity against one or more metazoan parasites, which antigen is characterised by possessing aspartyl proteinase-like and/or neutral endopeptidase-like activity and which in native form, is an integral membrane protein or protein complex.

A further major feature of the antigens of the invention is that they bind to pepstatin. Pepstatin is well known as an inhibitor of aspartyl proteases, and to a lesser degree, it is also documented to bind to membrane metallo-endopeptidases, including neutral endopeptidase.

Proteolytic activity in Haemonchus, Ostertagia and Trichostrongylus was found to be inhibited by pepstatin. A further aspect of the invention thus provides protective metazoan parasite antigens or antigenic fragments, components or precursors thereof and functionally-equivalent derivatives, analogues or variants thereof having protective antigenic activity against one or more metazoan parasites, which antigen is characterised by being able to bind to pepstatin and which, in native form, is an integral membrane protein or protein complex.

Isoelectric focusing studies have shown the isoelectric point (pI) of the glycoprotein antigens of the invention to lie in the region of about pH 2 to pH 7.5.

Polyacrylamide gel electrophoresis (PAGE) studies have been carried out on the antigens of the invention. Such studies have shown differing behaviour on reducing and non-reducing gels. In particular, larger bands on non-reducing gels break down to one or more smaller bands upon re-electrophoresis under reducing conditions. As will be described in more detail below, proteolytic and protective antigenic activity can be ascribed to the proteins of individual or composite bands, although certain bands can be more "active" than others. It may be that the antigens of the invention represent multimeric enzyme complexes.

In the case of the antigen H-gal-GP, the results of PAGE studies are described in detail in Example 1 below. Accordingly, a preferred subset of antigens according to the invention comprises the antigens (and their fragments or components) described in Example 1, and their functionally equivalent derivatives, analogues and variants, including analogues in other genera or species. Thus, preferred antigens according to the invention may comprise antigens, isolatable from Haemonchus or other metazoan parasite genera, having the SDS-PAGE molecular weights set out in Table 1.

In the case of the antigen H-gal-GP, protective antigenic activity has been shown to be associated with certain component protein bands in particular, as is described in more detail in Example 7, experiment 5, below.

Thus a still further aspect of the invention provides a protective metazoan parasite antigen or antigenic fragments, components or precursors thereof, and functionally-equivalent derivatives, analogues or variants thereof having protective antigenic activity against one or more metazoan parasites, which antigen is, or forms part of, an integral membrane protein or protein complex obtainable from the gut of Haemonchus sp. and has a molecular weight of 190 to 205 kd as determined by SDS-PAGE on a 5% gel under non-reducing conditions or 35 to 50 kd as determined by SDS-PAGE on a 10% gel under non-reducing conditions.

The metazoan parasites of particular concern in the present invention include especially helminths and arthropod parasites, in the case of the latter, particularly the blood-feeding insect parasites, cattle ticks and myiasis flies. As representative of such helminth and arthropod parasites may be mentioned for example, the various species listed above.

The term "protective antigen" as used herein defines those antigens capable of generating a host-protective, ie. immunogenic, immune response ie. a response by the host which leads to generation of immune effector molecules, antibodies or cells which damage, inhibit or kill the parasite and thereby "protect" the host or host species from clinical or sub-clinical disease and loss of productivity. Such a protective immune response may commonly be manifested by the generation of antibodies which are able to inhibit the metabolic function of the parasite, leading to stunting, lack of egg production and/or death.

In the case of certain metazoan parasites, notably the helminths, and those ectoparasites that are obligate feeders eg. lice and mites or those that feed for some period of time on the host eg. blowfly, warble fly, screw worm, and bot larvae, horn/buffalo flies and ticks, the host protective immune response may result in death, debilitation and/or expulsion of the parasite from the infected animal. In other words the parasitic burden on the infected animal is reduced and the infected animal itself directly reaps the benefit of its own immune response. In other cases however, an "epidemiological" effect may be observed whereby the immediate host may not necessarily itself benefit significantly from its immune response. For example as a result of feeding on immunised hosts, populations of parasites in a given area may be reduced and/or attenuated, thereby protecting later herds/flocks of host. Maintaining the immunisation approach through a number of seasons will continue to reduce the threat of parasite disease. This is particularly true for arthropod ectoparasites such as ticks, flies, etc. which may feed and move rapidly from host to hosts. In this way inhibitory antibodies ingested by the parasite may exert their effect after the parasite has left that host, but by killing, debilitating or reducing the fecundity of the parasite, other members of the host animal group may be protected.

As will be described in more detail below, protective, immunogenic, activity has been demonstrated for the antigens of the invention. It has further been shown that this protective activity is maintained when the antigens are denatured, for example by dissociation and/or reduction. Indeed, as mentioned above, certain components of the Haemonchus antigen complex H-gal-GP have been shown to be particularly protective.

Alternatively viewed, the invention can also be seen to provide use of a metazoan parasite antigen as hereinbefore defined, and components, fragments, precursors and functionally-equivalent derivatives, analogues or variants thereof having protective antigenic activity against one or more metazoan parasites, for the preparation of a vaccine composition for use in stimulating an immune response against metazoan parasites in a human or non-human, preferably mammalian, especially preferably ruminant, animal.

The invention also provides a vaccine composition for stimulating an immune response against metazoan parasites in a human or non-human, preferably mammalian, animal comprising one or more antigens as defined above, or components, fragments, precursors and functionally equivalent derivatives, analogues or variants thereof having protective antigenic activity against one or more metazoan parasites, together with a pharmaceutically acceptable carrier or diluent, and a method of stimulating an immune response against metazoan parasites in a human or non-human, preferably mammalian animal, comprising administering to said animal a vaccine composition as defined above.

As mentioned above, included within the scope of invention are functionally-equivalent derivatives, analogues and variants of the novel glycoprotein antigens. "Functionally equivalent" is used herein to define proteins related to or derived from the native enzyme proteins, where the amino acid sequence has been modified by single or multiple amino acid substitution, addition and/or deletion and also sequences where the amino acids have been chemically modified, including by deglycosylation or glycosylation, but which nonetheless retain protective antigenic activity eg. are capable of raising host protective antibodies and/or functional immunity against the parasites. Such functionally-equivalent variants may occur as natural biological variations or may be prepared using known techniques. For example functionally-equivalent recombinant proteins may be prepared using the known techniques of site-directed mutagenesis, random mutagenesis, or enzymatic cleavage and/or ligation of nucleic acids. As demonstrated in more detail in the Examples below with reference to the analogous antigen complexes H-gal-GP and O-gal-GP, such functionally-equivalent analogues may occur in different parasite species.

Antigens according to the invention may be obtained from a range of metazoan parasite species, including for example the nematodes Haemonchus, Ostertagia and Trichostrongylus. (For the avoidance of doubt, the term "Ostertagia" as used herein includes Teladorsagia sp.) Preferred are those antigens, so called "broad spectrum" antigens, which are capable of stimulating host protective immune responses against, in addition to the parasite from which they were isolated, a broad range of other parasites. Thus for example the Haemonchus glycoprotein complex H-gal-GP may confer protection against different species of the nematode Ostertagia and vice versa.

As mentioned above, one of the ways in which the antigens of the invention may exert their host protective effects is by raising inhibitory antibodies which inhibit the growth, maintenance and/or development of the parasite. Such antibodies and their antigen-binding fragments (eg. F(ab)₂, Fab and Fv fragments ie. fragments of the "variable" region of the antibody, which comprises the antigen binding site) which may be mono- or polyclonal, form a further aspect of the invention, as do vaccine compositions containing them and their use in the preparation of vaccine compositions for use in immunising hosts against parasites. Such inhibitory antibodies may be raised by use of idiotypic antibodies. Anti-idiotypic antibodies may be used as immunogens in vaccines.

The antigens according to the invention may be prepared by extraction from parasites eg. helminths using conventional biochemical and surgical techniques such as homogenisation, either of the whole parasite or just its intestine, followed by isolation of the desired enzyme by conventional purification techniques such as centrifugation, selective precipitation, chromatography and the like. A preferred process takes advantage of the fact that the target molecules have particular selective binding activities by using an affinity chromatography system in which specific ligands are immobilised on a solid phase matrix.

Thus the invention also provides a process for the preparation of a vaccine for use in immunising a human or non-human, preferably mammalian, animal against a metazoan parasite infection, said process comprising preparing an extract of said parasite containing at least one protective antigen as defined above, purifying said antigen therefrom by binding said antigen to an immobilized phase including a specific binding partner for the antigen and subsequently eluting said antigen from said immobilized phase. Suitable specific binding partners include substrate analogues, for example the inhibitor pepstatin, and oligosaccharide specific lectins, e.g. those which bind specifically to galactose, and in particular β-linked N-acetyl-galactosamine.

Indeed, our studies have shown that certain lectins are particularly useful as ligands in affinity chromatography for identifying glycoproteins with protective antigenic potential on the luminal surface of parasite intestines.

Thus, a further aspect of the present invention provides a method for the identification of protective antigens in a parasite, comprising subjecting an integral membrane fraction of the parasite to affinity chromatography using an immobilised phase carrying one or more lectins with specificity for N-acetyl-galactosamine.

Such lectins are preferably peanut or jacalin lectin, or other lectins having specificity for β-linked N-acetyl-galactosamine.

An integral membrane fraction of the parasite can readily be prepared using techniques well known in the art eg. detergent extraction for example with Triton® X-100.

Affinity chromatography techniques are likewise well known in the art, and include batch and column-based systems. A range of solid phases to which the lectins may be attached, may be used eg. gels such as agarose or sepharose. Indeed, agarose beads carrying peanut or jacalin lectins are commercially available (Vector Laboratories).

Alternatively the antigens may be prepared by recombinant DNA technology using standard techniques, such as those described for example by Sambrook et al., 1989, (Molecular Cloning, a laboratory manual 2nd Edition, Cold Spring Harbor Press).

Nucleic acid molecules comprising a nucleotide sequence encoding the antigens of the invention form further aspects of the invention.

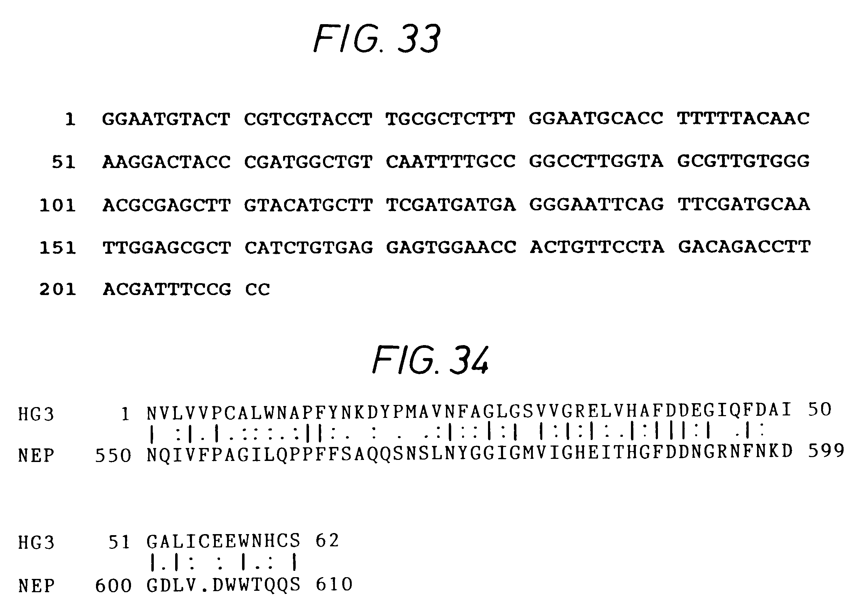

Preliminary sequence studies of clones corresponding to the antigen H-gal-GP have been performed. The sequence of one such clone, designated HG3, is shown in Figure 33 (SEQ ID NO: 1). This sequence shows significant homology with known mammalian neutral endopeptidase enzyme sequences as demonstrated by the amino acid alignment shown in Figure 34 (SEQ ID NO: 2).

Accordingly, a preferred nucleic acid molecule according to the invention comprises one or more nucleotide sequences substantially corresponding to all or a portion of the nucleotide sequence shown in Figure 33 (SEQ ID NO: 1) or a sequence which is degenerate or substantially homologous with or which hybridises with said sequence.

Nucleic acid molecules according to the invention may be single or double stranded DNA, cDNA or RNA, preferably DNA, and include degenerate, substantially homologous and hybridising sequences which are capable of coding for the antigen or antigen fragment concerned. By "substantially homologous" is meant sequences displaying at least 60%, preferably at least 70% or 80% sequence homology. Hybridising sequences included with the scope of the invention are those binding under non-stringent conditions (6 x SSC/50% formamide at room temperature) and washed under conditions of low stringency (2 x SSC, room temperature, more preferably 2 x SCC, 42°C) or conditions of higher stringency eg. 2 x SSC, 65°C (where SSC = 0.15M NaCl, 0.015M sodium citrate, pH 7.2), as well as those which, but for the degenerary of the code, would hybridise under the above-mentioned conditions.

Derivatives of nucleotide sequences capable of encoding antigenically active antigens or antigen variants according to the invention may be obtained by using conventional methods well known in the art.

Antigens according to the invention may be prepared in recombinant form by expression in a host cell containing a recombinant DNA molecule which comprises a nucleotide sequence as broadly defined above, operatively linked to an expression control sequence, or a recombinant DNA cloning vehicle or vector containing such a recombinant DNA molecule. Synthetic polypeptides expressed in this manner form a further aspect of this invention (the term "polypeptide" is used herein to include both full-length protein and shorter length peptide sequences).

The antigen so expressed may be a fusion polypeptide comprising all or a portion of an antigen according to the invention and an additional polypeptide coded for by the DNA of the recombinant molecule fused thereto. This may for example be β-galactosidase, glutathione-S-transferase, hepatitis core antigen or any of the other polypeptides commonly employed in fusion proteins in the art.

Other aspects of the invention thus include cloning and expression vectors containing the DNA coding for an antigen of the invention and methods for preparing recombinant nucleic acid molecules according to the invention, comprising inserting nucleotide sequences encoding the antigen into vector nucleic acid, eg. vector DNA. Such expression vectors include appropriate control sequences such as for example translational (eg. start and stop codons, ribosomal binding sites) and transcriptional control elements (eg. promoter-operator regions, termination stop sequences) linked in matching reading frame with the nucleic acid molecules of the invention.

Vectors according to the invention may include plasmids and viruses (including both bacteriophage and eukaryotic viruses) according to techniques well known and documented in the art, and may be expressed in a variety of different expression systems, also well known and documented in the art. Suitable viral vectors include baculovirus and also adenovirus and vaccinia viruses. Many other viral vectors are described in the art.

A variety of techniques are known and may be used to introduce such vectors into prokaryotic or eukaryotic cells for expression, or into germ line or somatic cells to form transgenic animals. Suitable transformation or transfection techniques are well described in the literature.

The invention also includes transformed or transfected prokaryotic or eukaryotic host cells, or transgenic organisms containing a nucleic acid molecule according to the invention as defined above. Such host cells may for example include prokaryotic cells such as E.coli, eukaryotic cells such as yeasts or the baculovirus-insect cell system, transformed mammalian cells and transgenic animals and plants. Particular mention may be made of transgenic nematodes (see for example Fire, 1986, EMBO J., 5. 2673 for a discussion of a transgenic system for the nematode Caenorhabditis).

A further aspect of the invention provides a method for preparing an antigen of the invention as hereinbefore defined, which comprises culturing a host cell containing a nucleic acid molecule encoding all or a portion of said antigen, under conditions whereby said antigen is expressed and recovering said antigen thus produced.

The antigens of the invention and functionally equivalent antigen variants may also be prepared by chemical means, such as the well known Merrifield solid phase synthesis procedure.

Water soluble derivatives of the novel antigens discussed above form a further aspect of the invention. Such soluble forms may be obtained by proteolytic digestion, for example using the enzyme elastase. Generally speaking enzymic digestion of the antigens yields two fractions, a detergent soluble fraction (which remains with the membrane) and a water-soluble fraction.

A vaccine composition may be prepared according to the invention by methods well known in the art of vaccine manufacture. Traditional vaccine formulations may comprise one or more antigens or antibodies according to the invention together, where appropriate, with one or more suitable adjuvants eg. aluminium hydroxide, saponin, quil A, or more purified forms thereof, muramyl dipeptide, mineral or vegetable oils, Novasomes or non-ionic block co-polymers or DEAE dextran, in the presence of one or more pharmaceutically acceptable carriers or diluents. Suitable carriers include liquid media such as saline solution appropriate for use as vehicles to introduce the peptides or polypeptides into an animal or patient. Additional components such as preservatives may be included.

An alternative vaccine formulation may comprise a virus or host cell eg. a microorganism (eg. vaccinia virus, adenovirus or salmonella) which may be live, killed or attenuated, having inserted therein a nucleic acid molecule (eg. a DNA molecule) according to this invention for stimulation of an immune response directed against polypeptides encoded by the inserted nucleic acid molecule.

Administration of the vaccine composition may take place by any of the conventional routes, eg. orally or parenterally such as by intramuscular injection, optionally at intervals eg. two injections at a 7-35 day interval.

The antigens may be used according to the invention in combination with other protective antigens obtained from the same or different parasite species. Thus a vaccine composition according to the invention may comprise one or more of the antigens defined above e.g. H-gal-GP together with the antigens H110D and H45 mentioned above. Such a combined vaccine composition may contain smaller amounts of the various antigens than an individual vaccine preparation, containing just the antigen in question.

Animals which may benefit from the present invention may be any human or non-human animal, but companion animals, particularly dogs and cats and domestic animals, especially ruminants are preferred. Particular mention may be made of sheep, cattle, pigs, deer and goats.

The invention will now be discussed in more detail with particular reference to antigens from H. contortus, O.ostertagi and O.circumcincta which will be referred to as H-gal-GP, Oo-gal-GP and Oc-gal-GP respectively. While not limiting the generality of the invention as defined above, it will be understood that the use of H-gal-GP, Oo-gal-GP and Oc-gal-GP and/or the separate components thereof, in the preparation of helminth vaccines and the antigens and vaccines per se, form particularly preferred aspects of the invention.

The protein H-gal-GP is an integral membrane protein within the brush border lining of Haemonchus intestinal cells. It is glycosylated and binds selectively to lectins which have a specificity for N-acetylgalactosamine. It shows protease activity which can be inhibited by pepstatin, a specific inhibitor of aspartyl proteases. Under reducing conditions on SDS-polyacrylamide gels it runs as 8 major bands. For these reasons it has been named Haemonchus galactose-containing glycoprotein complex or H-gal-GP.

In the following non-limiting Examples, the Figures represent:

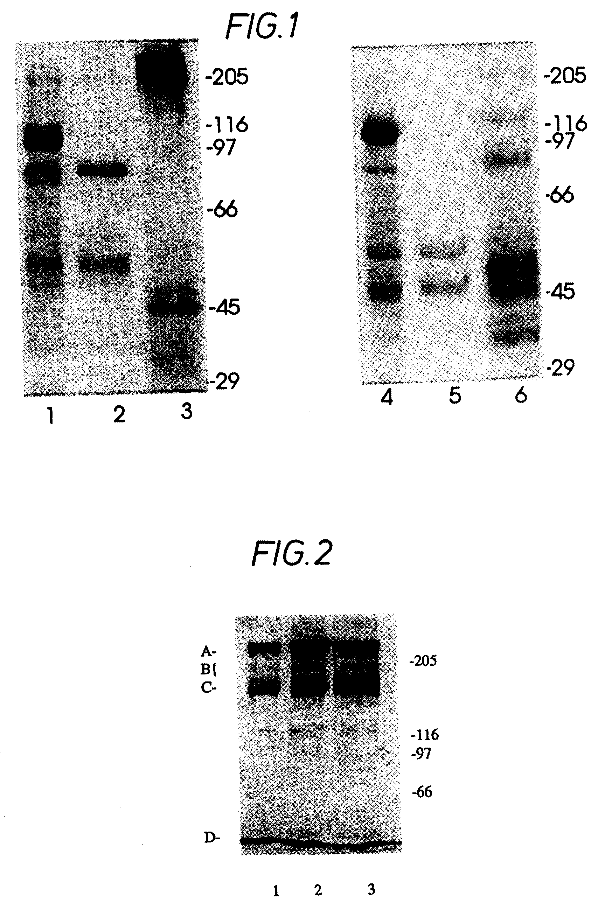

Figure 1 shows a comparison by SDS-PAGE of H-gal-GP, H110D and H-45 complex on 10% acrylamide gels.
   - Tracks 1 to 3 and 4 to 6: non-reduced and reduced samples, respectively.
   - Tracks 1 and 4: a fraction enriched for a mixture of H11 and H 45 complex
   - Tracks 2 and 5: H 45 complex
   - Tracks 3 and 6: H-gal-GP
   The N terminal amino acid sequences of the 47 and 50 kd bands in track 3 were determined;
Figure 2 shows an SDS-PAGE profile of H-gal-GP on 5% gels under non-reducing conditions. Each track contains a different loading of H-gal-GP. Letters show the bands excised for antigen in sheep experiment 5 (see Example 7 and Figure 22), as follows:
   A = 230 kd for Group 15.
   B = 190-205 kd diffuse band for Group 16.
   C = 170 kd for Group 17.
   D = dyefront bands for Group 18;
Figure 3 shows an analysis, under reducing conditions of major H-gal-GP bands previously separated under non-reducing conditions:
   - Track 1: H-gal-GP
   - Track 2: 230 kd ) bands of these apparent molecular
   - Track 3: 170 kd ) weight were excised from a 5%
   - Track 4: dyefront ) non-reducing gel (e.g. Fig 2)
   ) and re-electrophoresed reduced
   The N-terminal amino acid sequence of the 45 and 48 Kd bands in track 3 was determined;

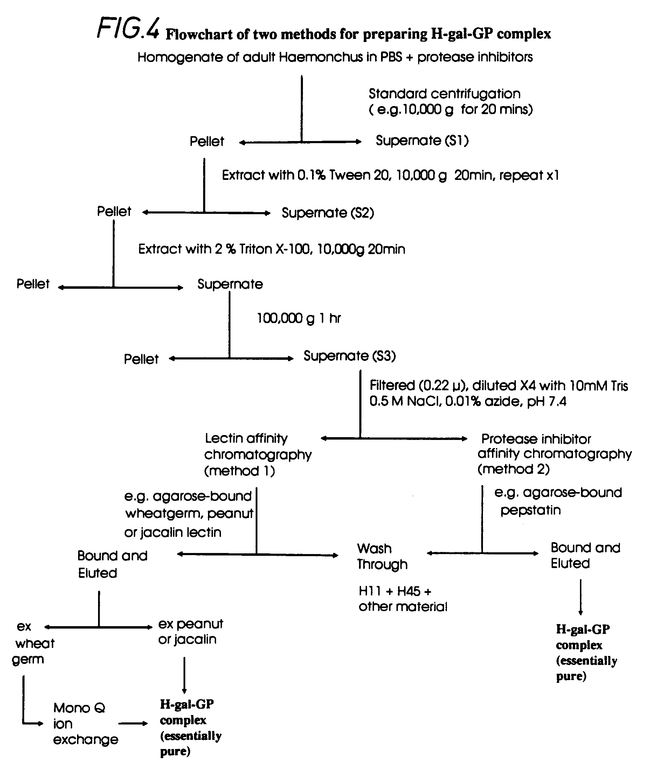

Figure 4 shows a flowchart of three methods for preparing H-gal-GP complex;

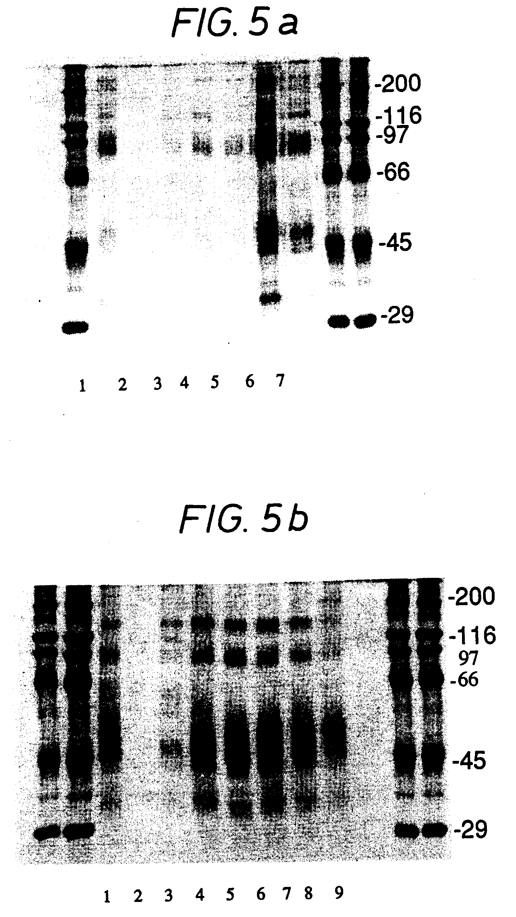

Figure 5 shows SDS-PAGE (10 % gel, reducing) of fractions obtained following ion exchange chromatography of H-gal-GP complex on Mono Q medium:
   a) start material previously isolated by peanut lectin
      - Track 1: Start material applied to column
      - Track 2: Unbound fraction
      - Tracks 3-5: Eluted with 140 mM NaCl
      - Tracks 6 and 7: Eluted with 400 mM NaCl;
   b) start material previously isolated by jacalin lectin
      - Track 1: Start material applied to column
      - Track 2: Unbound fraction
      - Tracks 3-5: Eluted with 140 mM NaCl
      - Tracks 6 and 7: Eluted with 400 mM NaCl
      - Track 8: Eluted with 500 mM NaCl
      - Track 9: Eluted with 1M NaCl;

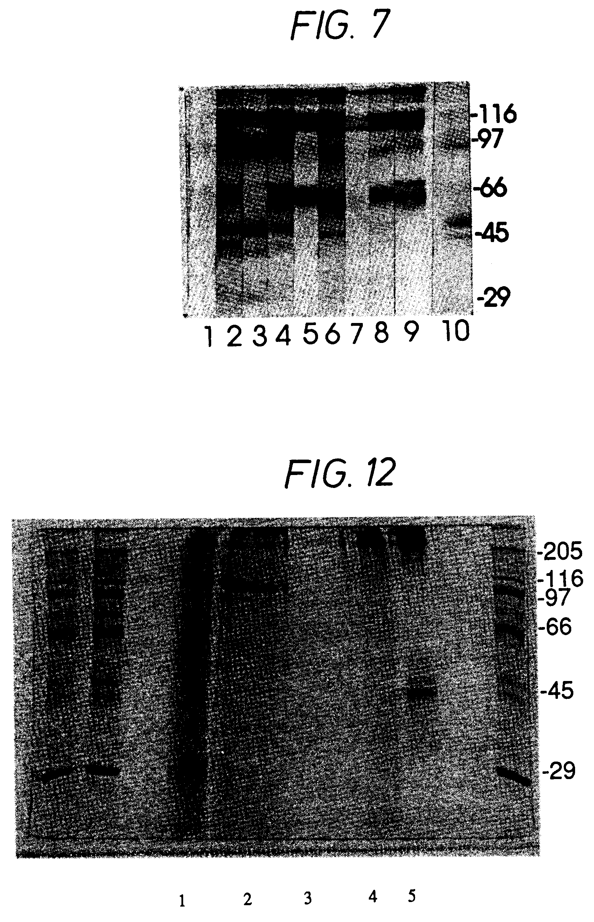

Figure 6 shows a comparison by SDS-PAGE of the components within a Triton® X-100 extract of H.contortus membranes which bound to either peanut, Vicia, Lotus, Soybean or Dolichos lectins.
   Identical aliquots of a Triton® X-100 extract of H.contortus membranes were passed through columns containing an excess of the following agarose-coupled lectins:- 1) peanut, 2) Vicia villosa, 3) Lotus tetranoglobulus, 4) Soybean or 5) Dolichos biflorus. Bound material was eluted with the appropriate sugar (Table 2) and loaded without reduction on a 5% acrylamide gel with tracks numbered in the same sequence;
Figure 7 shows the binding of H-gal-GP subcomponents to a panel of biotinylated lectins. H-gal-GP was electrophoresed on 10 % gels under reducing conditions and blotted to immobilon membrane. The blot was cut into numbered strips which were then respectively incubated with the following biotinylated lectins:- 1 none (negative control), 2 concanavalin A, 3 lentil, 4 wheatgerm, 5 peanut, 6 jacalin, 7 soybean, 8 succinylated wheatgerm, 9 helix pomatia. Strip 10 was stained for protein with Coomassie blue.

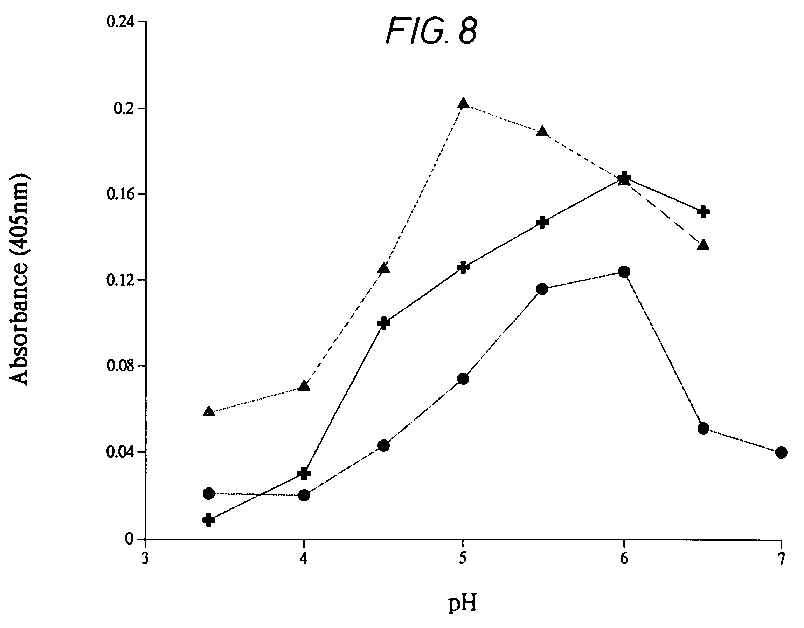

Figure 8 shows pH optima with Azocasein as substrate; (Abscissa shows pH, ordinate shows Absorbance at 405 nm: ● H-gal-GP, ▲ Oc-gal-GP, + Oo-gal-GP);

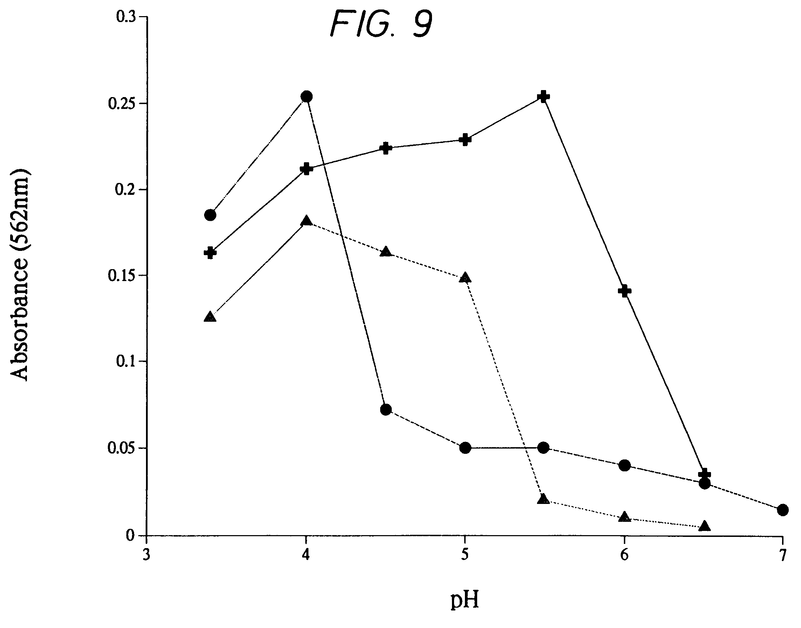

Figure 9 shows pH optima with Haemoglobin as substrate; (Abscissa shows pH, ordinate shows Absorbance at 562 nm: ● H-gal-GP, ▲ Oc-gal-GP, + Oo-gal-GP);

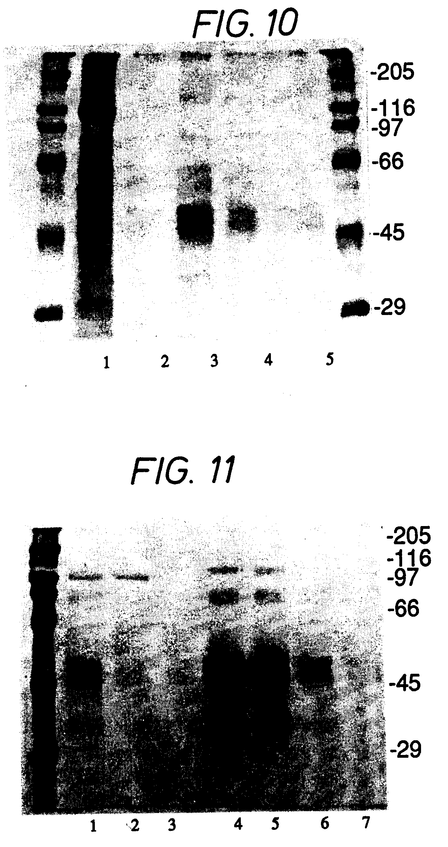

Figure 10 shows binding of H-gal-GP complex to Peanut lectin (10% gel, reducing conditions)
   - Track 1: S3 extract of Haemonchus applied to peanut lectin column
   - Tracks 2-5: Fractions of peak eluted from column by 0.3M galactose;
Figure 11 shows binding of H-gal-GP complex to Jacalin lectin (10% gel, reducing conditions)
   - Track 1: Material applied to jacalin lectin column. (A fraction of Haemonchus S3 which bound and was eluted from wheat germ lectin)
   - Track 2: Unbound fraction = enriched for H110D
   - Tracks 4-7: Fractions over peak eluted from column by 0.8M galactose.
   Note: A major band in the starting material is H110D. This does not bind to jacalin and can just be distinguished under these conditions as being slightly smaller than the 130kd component of H-gal-GP which does;

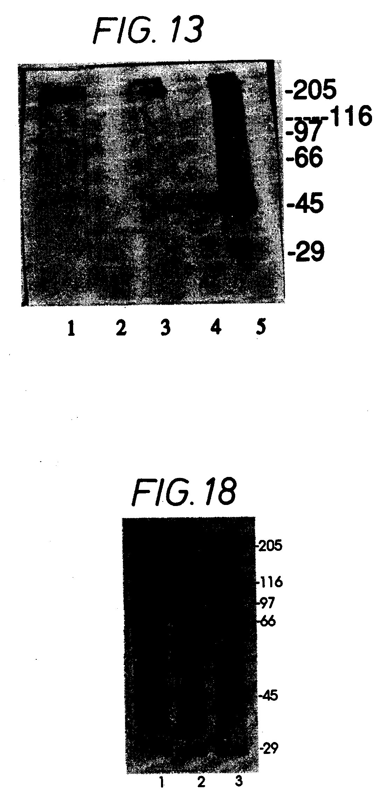

Figure 12 shows selective binding of H-gal-GP to pepstatin agarose (10% gel, non-reducing conditions)
   - Track 1: Haemonchus S3 applied to pepstatin agarose at low pH (5.5)
   - Track 2: Unbound material rich in H110D
   - Track 3: Material eluted at pH 8.5
   - Track 4: Material eluted with 1% SDS, pH 7.4
   - Track 5: H-gal-GP (positive control);
Figure 13 shows selective binding of H-gal-GP to pepstatin agarose and the avidity of the 47 and 50 kd bands (immunoblot from a 10% gel run under non-reducing conditions probed with anti H-gal-GP serum)
   - Track 1: Unbound material rich in H110D
   - Track 2: Material eluted at pH 8.5
   - Track 3: Material eluted with 1% SDS, pH 7.4
   - Track 4: Material still bound to pepstatin agarose after 1% SDS elution step (-released by unpacking column and boiling agarose with SDS PAGE sample buffer containing SDS and DTT)
   - Track 5: H-gal-GP (positive control);

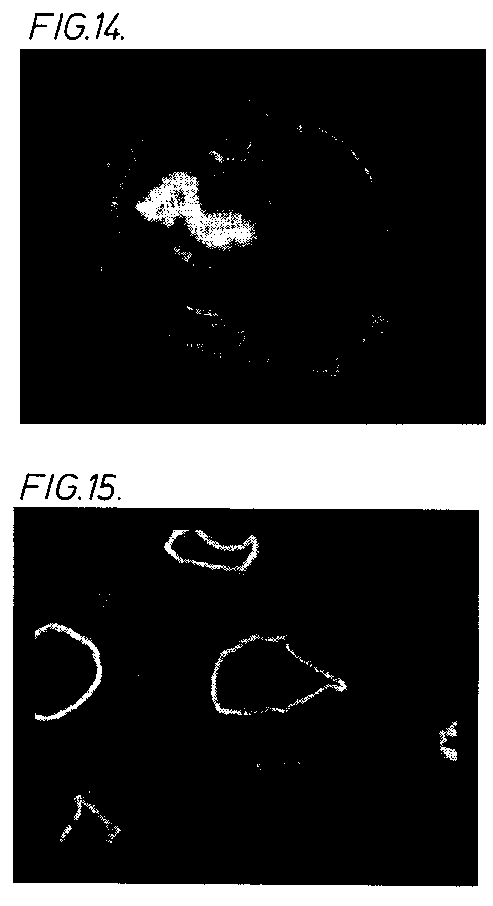

Figure 14 shows a cryostat section of H.contortus stained with fluorescent labelled peanut lectin;
Figure 15 shows cryostat sections of H.contortus stained with fluorescent labelled wheatgerm lectin;

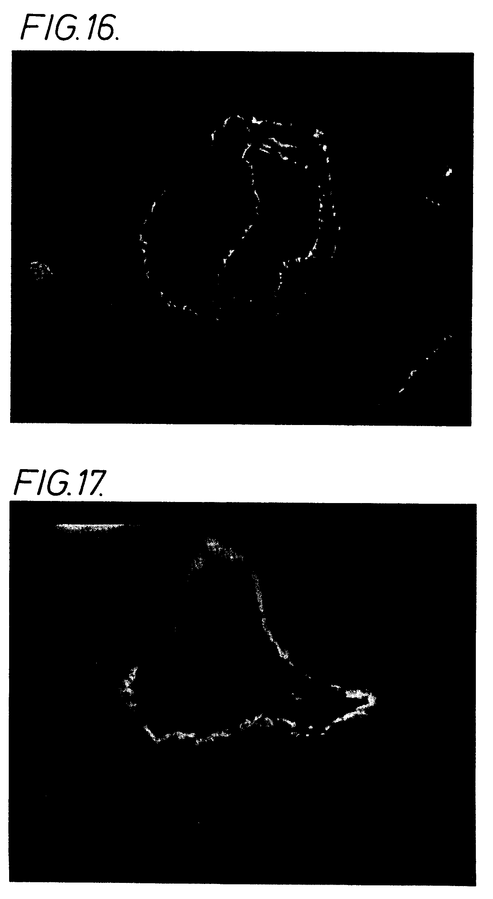

Figure 16 shows cryostat sections of H.contortus stained with anti H-gal-GP serum;
Figure 17 shows a cryostat section of H.contortus recovered from a sheep immunised with H-gal-GP and stained with antiserum specific for ovine immunoglobulin;

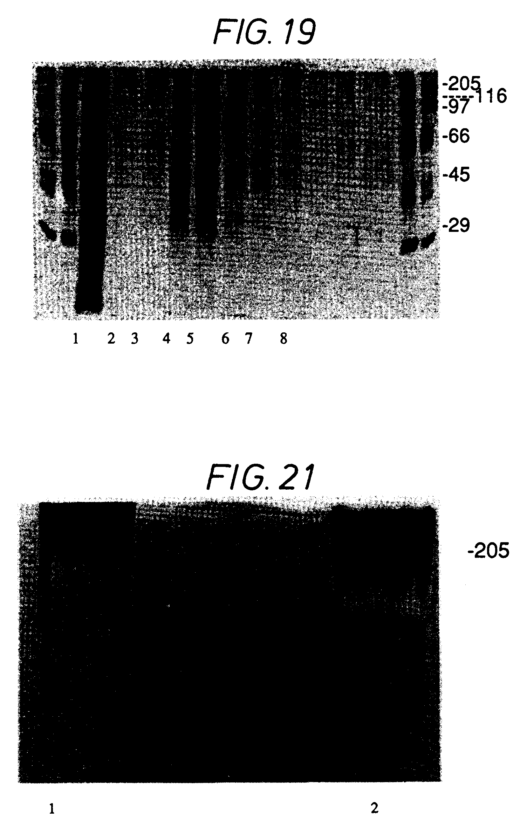

Figure 18 shows the distribution of H-gal-GP within PBS (S1), Tween® (S2) and Triton® X-100 (S3) extracts of H.contortus membranes;
   Tracks 3, 2 and 1 contain sequential PBS, Tween® and Triton® X- 100 extracts from the same batch of worms. The samples were reduced, electrophoresed on 10% gels, blotted, periodate treated to denature carbohydrate groups and probed with antiserum to H-gal-GP;
Figure 19 shows the binding of H-gal-GP and H110D to wheatgerm lectin (10% gel, reducing conditions)
   - Track 1: S3 extract of Haemonchus applied to the column
   - Tracks 2-8: Sequential fractions, enriched for H110D and H-gal-GP, eluted over the peak with 0.5M N-acetylglucosamine;

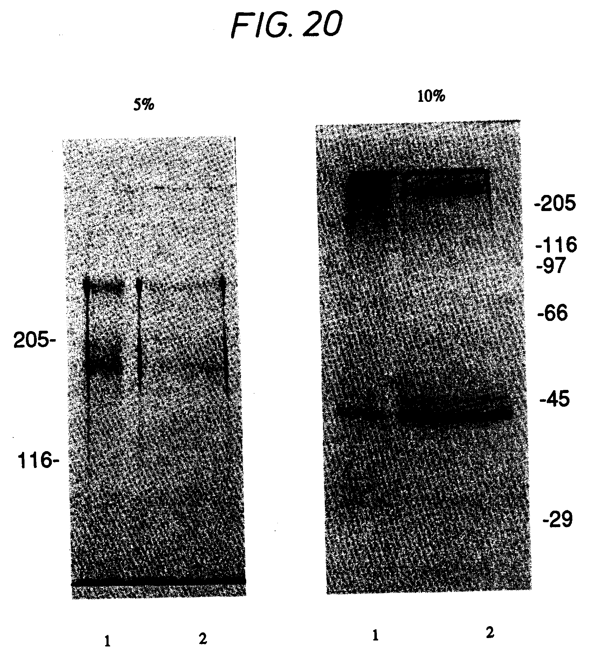

Figure 20 shows binding of H-gal-GP to pepstatin agarose (5% or 10% acrylamide gels, non-reducing conditions).
   - Track 1: H-gal-GP applied to the pepstatin agarose column
   - Track 2: Material desorbed from the column with 1% SDS in 10mM Tris-HCl, 50mM NaCl pH 8.0;

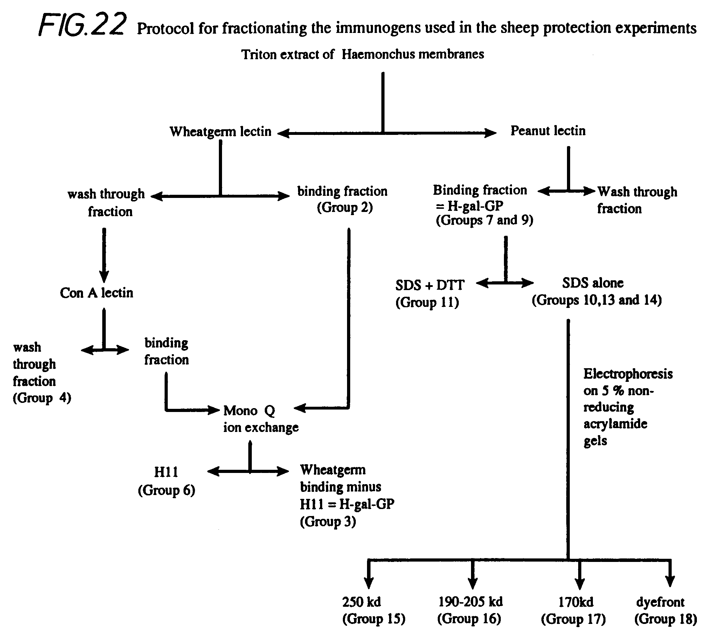

Figure 21 shows a substrate gel analysis of the hydrolytic activity of H-gal-GP. Close up view of a 7.5% non-reducing acrylamide gel containing 0.1% gelatin as substrate.
   - Track 1: H-gal-GP preincubated with pepstatin
   - Track 2: H-gal-GP untreated;
Figure 22 shows the protocol for fractionating the immunogens used in the sheep protection experiments described in Example 7;

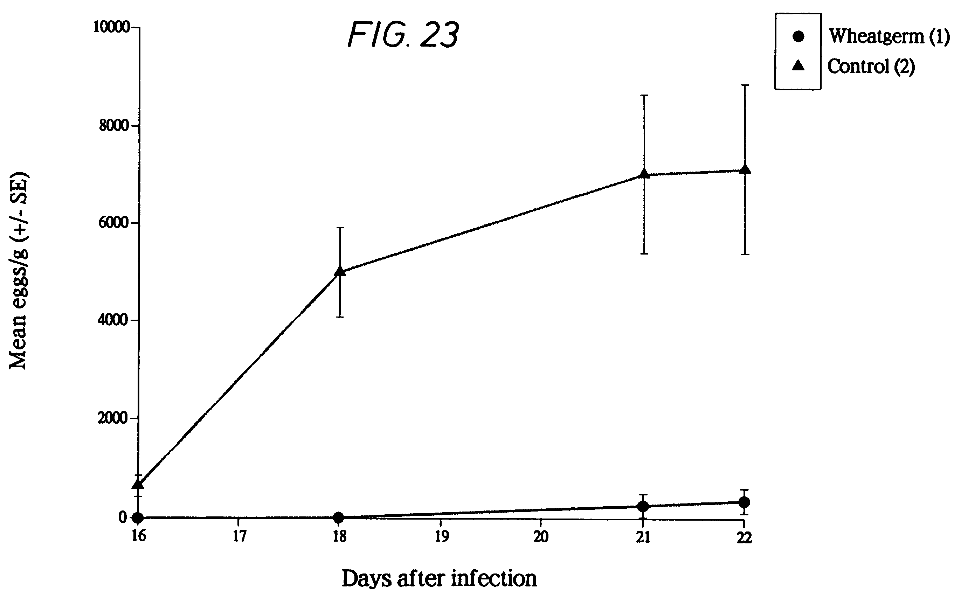

Figure 23 shows the results of Experiment 1 of Example 7: group mean faecal egg counts ● Wheatgerm (1), ▲ Control (2) (Abscissa shows days after infection; ordinate shows mean eggs/g (+/- SE));

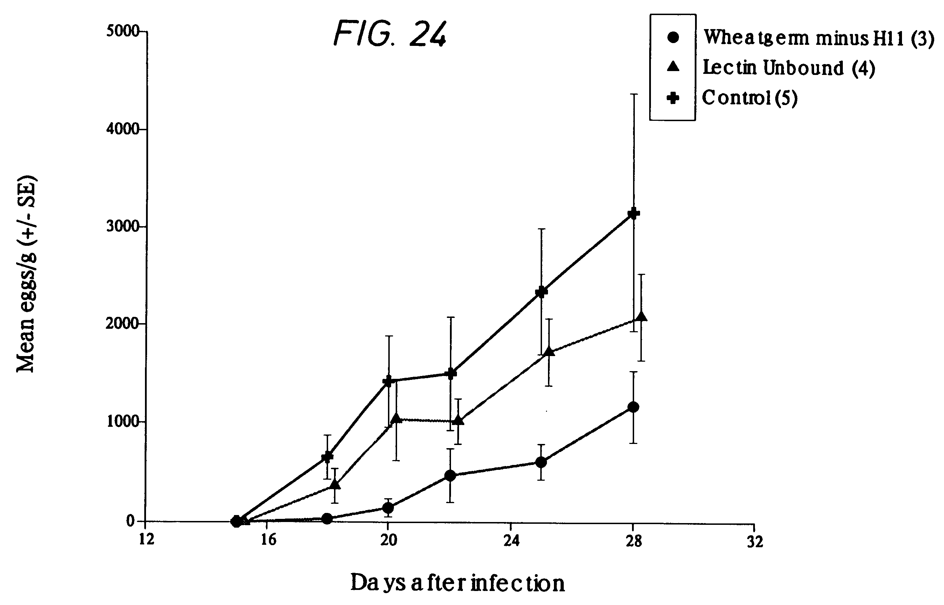

Figure 24 shows the results of Experiment 2 of Example 7: group mean faecal egg counts ● Wheatgerm depleted of H11 (3), ▲ Lectin Unbound (4), + Control (5) (Abscissa shows days after infection; ordinate shows mean eggs/g (+/- SE));

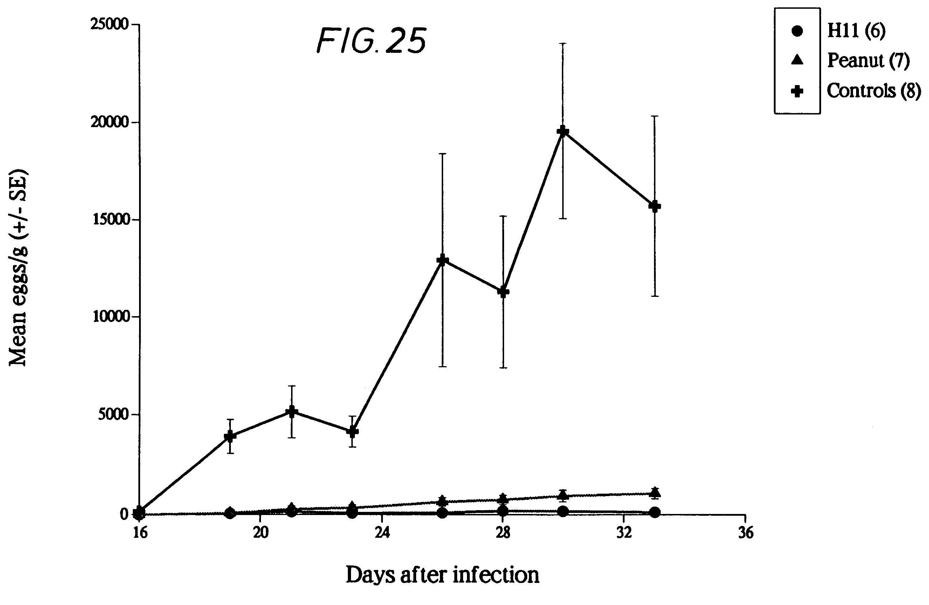

Figure 25 shows the results of Experiment 3 of Example 7: group mean faecal egg counts ● H110D (6), ▲ Peanut (7), + Controls (8) (Abscissa shows days after infection; ordinate shows mean eggs/g (+/- SE));

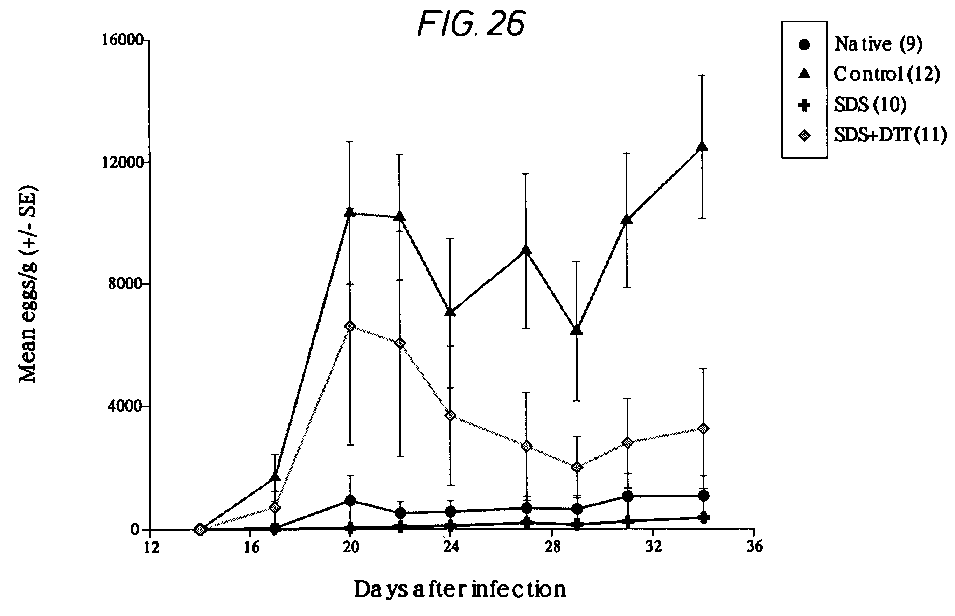

Figure 26 shows the results of Experiment 4: group mean faecal egg counts ● Native (9), ▲ Control (12), + SDS (10), ◇ SDS+DTT (11) (Abscissa shows days after infection; ordinate shows mean eggs/g (+/- SE));

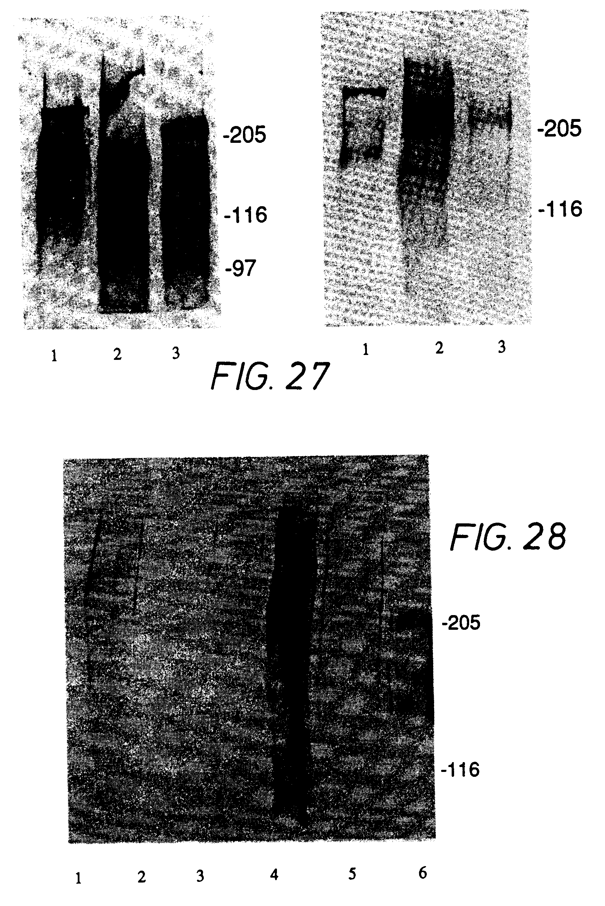

Figure 27 shows the similarities between H-gal-GP and equivalent proteins from O.circumcincta and O.ostertagi. The samples were electrophoresed on 5% non-reducing acrylamide gels and blotted to immobilon membrane. The blot on the left was probed with biotinylated peanut lectin, the one on the right with anti H-gal-GP serum. The blot probed with antiserum was preincubated with 50mM periodate to denature potentially cross-reactive carbohydrate epitopes. No staining was observed on a control blot incubated with normal serum.
   - Track 1: H-gal-GP
   - Track 2: O.circumcincta-gal-GP
   - Track 3: O.ostertagi-gal-GP;
Figure 28 shows the binding of O.circumcincta-gal-GP to pepstatin agarose;
   Equal quantities of O.circumcincta-gal-GP were added to tubes containing agarose beads coupled to either pepstatin, peanut lectin or dolichos lectin and the tubes were gently agitated for 3 hours at 4C. The beads were pelleted and the unbound supernates were retained.
   After three washes the beads were boiled in a minimum volume of SDS PAGE sample buffer and pelleted. The supernates from this step, containing material which had bound to the agarose, together with the unbound supernates were run on non-reducing 5% SDS PAGE gels, blotted to immobilon membrane and probed with biotinylated peanut lectin.
   The result showed that O.circumcincta-gal-GP bound to peanut lectin and to pepstatin agarose specifically because it did not bind to agarose coupled with an alternative ligand (dolichos lectin).
   - Track 1: Dolichos unbound
   - Track 2: Dolichos bound
   - Track 3: Peanut unbound
   - Track 4: Peanut bound
   - Track 5: Pepstatin unbound
   - Track 6: Pepstatin bound;

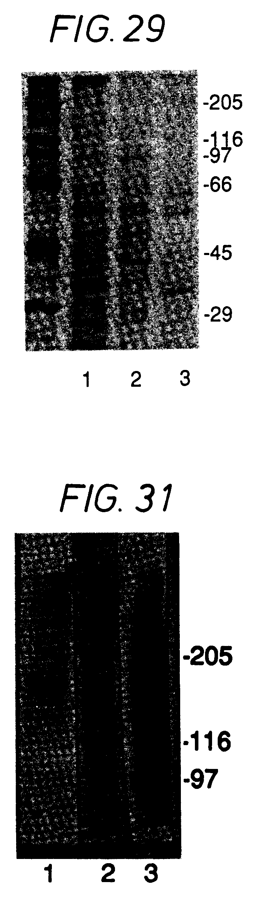

Figure 29 shows an SDS-PAGE profile (10% acrylamide gel; reducing conditions) of an antigen preparation of integral membrane proteins of O. circumcincta, used in a subsequent immunisation experiment.
   - Track 1: Triton® X-100 extract of O.circumcincta membranes from whole worms (S3).
   - Track 2: ConA binding fraction of S3 used to immunise the sheep in Example 8, Experiment 1
   - Track 3: Fraction of S3 which did not bind to ConA
   The remaining track contains molecular weight markers;

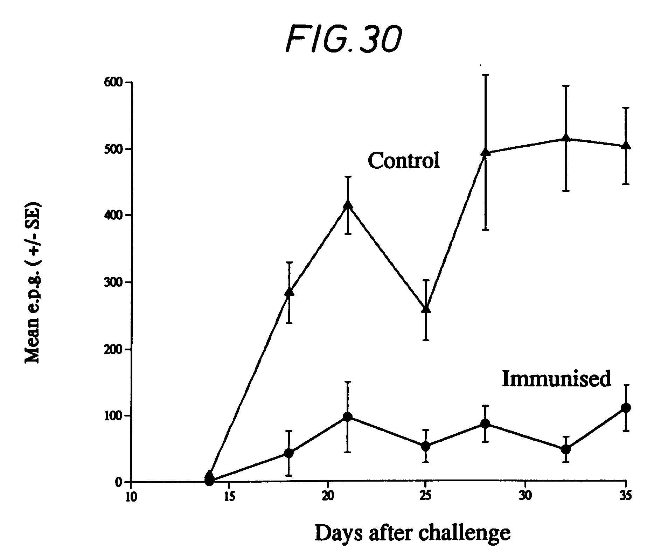

Figure 30 shows the results, in terms of group mean faecal egg counts, following challenge with 5000 O. circumcincta larvae in control lambs and lambs immunised with a concanavalin A (ConA) binding fraction of O. circumcincta integral membrane proteins (abscissa shows days after challenge; ordinate shows group mean faecal egg counts, mean e.p.g. (+/- SE));

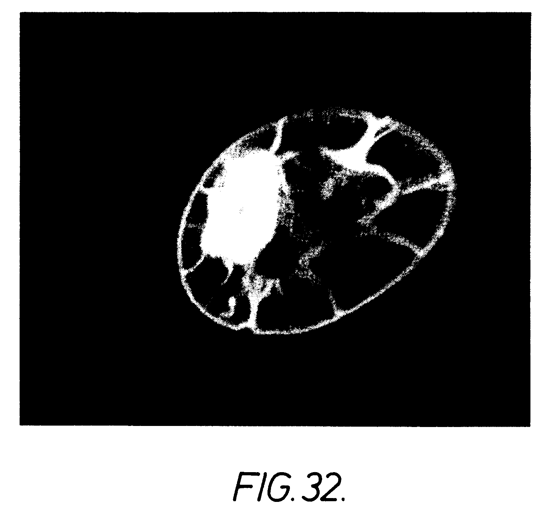

Figure 31 shows blots (5% acrylamide gel; non-reducing conditions) of the ConA binding fraction from Example 8, Experiment 1, showing the presence of Oc-gal-GP.
   - Track 1: ConA S3 fraction of O. circumcincta probed with peanut lectin
   - Track 2: Molecular weight markers
   - Track 3: ConA S3 fraction of O. circumcincta probed with ConA lectin
Figure 32 shows fluorescence staining of cryostat sections of normal adult O. circumcincta with fluorescein conjugated peanut lectin;
Figure 33 shows the nucleotide sequence of clone HG3;
Figure 34 shows the derived amino acid sequence of clone HG3 and an alignment to human neutral aminopeptidase. Both sequences are deduced from cDNA. The degree of amino acid identity is 33%, the level of similarity is 65%. The alignment was produced using the BESTFIT program from the University of Wisconsin GCG package;

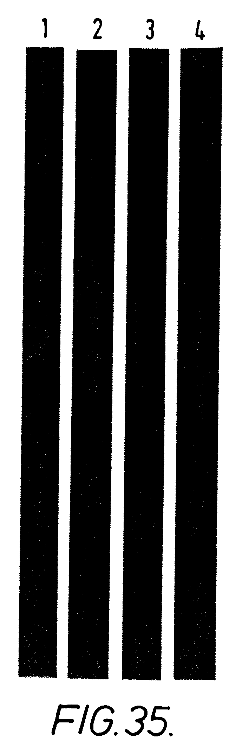

Figure 35 shows a southern plot demonstrating the hybridisation of clone HG11 to genomic DNA derived from various metazoan parasites and digested with Eco RI. Hybridisation was performed under conditions of moderate stringency.
   - Track 1: Haemonchus
   - Track 2: Lucilia
   - Track 3: Nematodirus
   - Track 4: Boophilus

### EXAMPLE 1

### Description and biochemical characterisation of H-gal-GP complex

### 1) SDS-PAGE profiles.

H-gal-GP complex used for this analysis was isolated from a Triton® X-100 extract of adult Haemonchus (as detailed in Example 2 and Fig. 4 below) by peanut lectin affinity chromatography followed, after a desalting step, by concentration using anion exchange chromatography with Mono Q medium (Pharmacia). The fraction eluted between 100 and 350 mM NaCl was retained. A typical yield was approximately 2% of the total protein in the Triton® X-100 extract, equivalent to some 200 µg of protein per g of worms.

When H-gal-GP complex was electrophoresed on 7.5 or 10% gels without reduction, the protein profile consisted of a group of polypeptides at about 200 kd or greater together with a lighter staining pair of bands of about 47 and 50 kd (Fig. 1, Track 3). However, if the acrylamide concentration was lowered to 5%, the high molecular weight material resolved as two main bands of about 230 and 170 kd (Fig. 2). These straddled a fainter 205 kd polypeptide and a diffuse staining area (Fig. 2). The smaller polypeptides seen in Fig. 1, Track 3 ran unresolved at or near the dyefront.

Under reducing conditions unfractionated H-gal-GP complex resolved into some 8 bands on 10% acrylamide gels. Most of the protein resolved as 3 bands between 45 and 50 kd, but fainter bands were also visible at about 33, 35, 37, 90 and 130 kd (Fig. 1, Track 6).

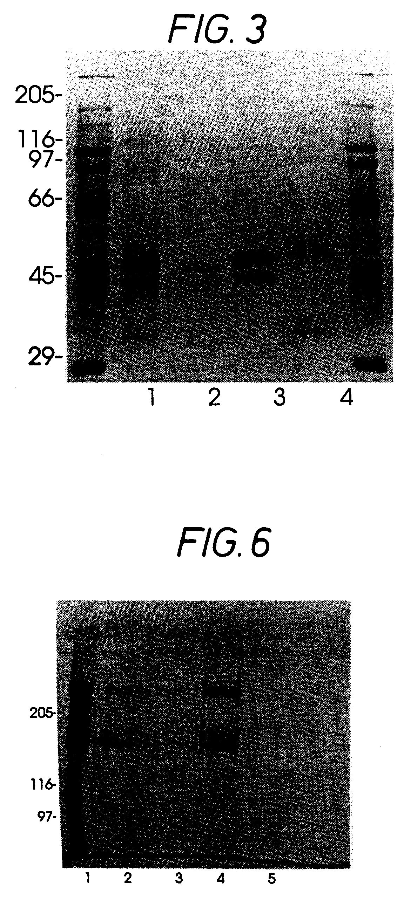

Further analysis of the composition of the unreduced polypeptides, which is summarised in Table 1, was made by excising the 3 main bands from 5% non-reducing gels (i.e. at 230 kd, 170 kd and the dyefront of e.g. Fig. 2) and, after electroelution and concentration, re-electrophoresing them on 10% acrylamide under reducing conditions (Fig. 3). The 170 kd band clearly reduced into 2 heavily staining components of about 45 and 48 kd (Fig 3, Track 3). The largest band (230 kd) was more complex and separated into about 5 subcomponents with molecular weights of circa 130, 90, 48, 40 and 33 kd (Fig. 3, Track 2). The "dyefront" bands resolved into 3 components of approximately 50, 47 and 35 kd (Fig. 3, Track 4). The first two of these were also described above in Fig. 1, Track 3 when the whole H-gal-GP complex was electrophoresed on 7.5 or 10% gels without reduction.

The proteins of the above bands all represent components of H-gal-GP according to the invention.

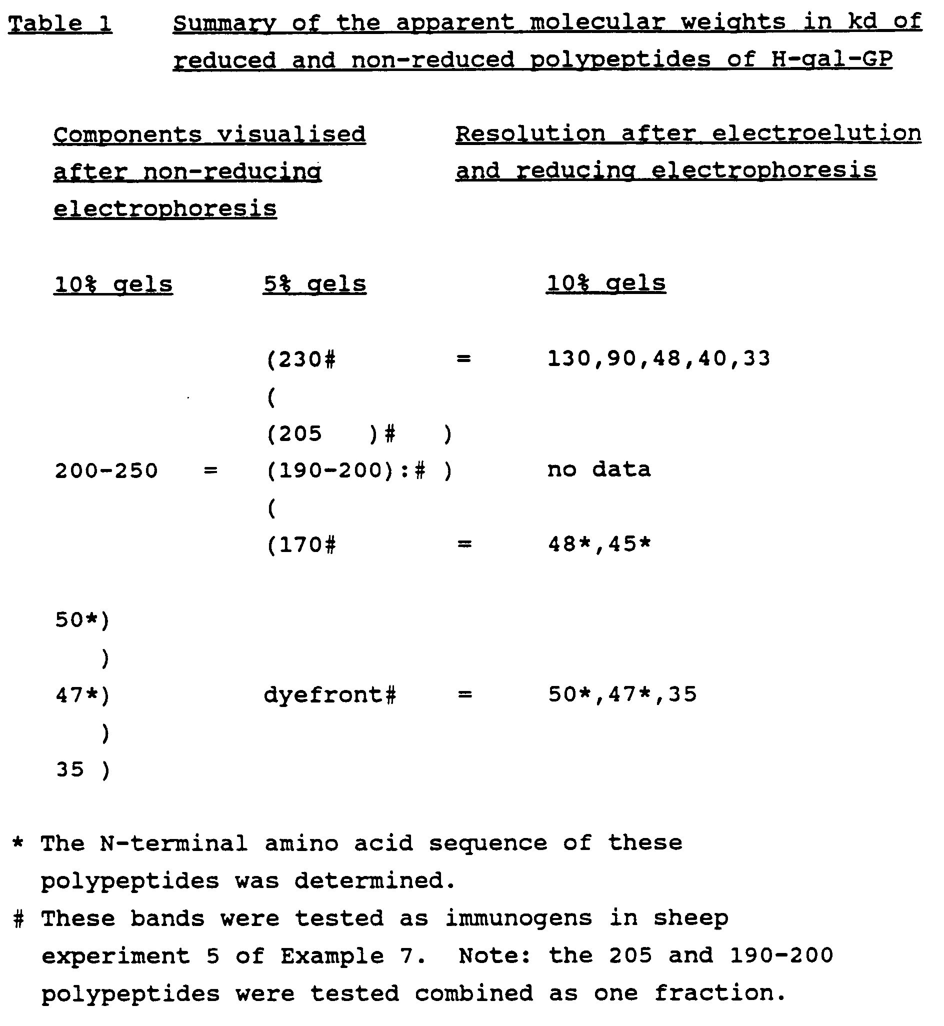

Therefore H-gal-GP comprises a minimum of 10 distinct components as well as the 205 and 190-200 kd material seen under non-reducing conditions.

### 2) N-terminal amino acid sequences of four of the bands.

Gels containing tracks identical to No. 3, Fig. 3 and No. 3, Fig. 1 were prepared and electrophoresed onto membranes (ProBlott - Applied Biosystems). After being stained with Coomassie Blue, strips containing the pairs of polypeptides identified in Figs. 1 and 3 were excised from the membranes and the N-terminal amino acid composition of each was determined on an Applied Biosystems Model 477A Peptide sequencer to yield the following sequences:-
- Fig. 3, Track 3: 45 kd : Val-Asp-Asn-Val-Phe-?-Pro-Asn-Val-Gly-Leu. (SEQ ID NOS: 3 and 4) 48 kd : Val-Glu-Arg-Thr-Asp-Ala-Arg-Met-Asn-(ie -Glu- or -Asn-) (SEQ ID NOS: 5 and 6)
- Fig. 1, Track 3: 47 kd : Val-Phe-Pro-His-Pro-Ile-Tyr-Asp-Tyr-Gln (SEQ ID NO: 7) 50 kd : Ala-?-Gln-Thr-Val-Phe-Pro-His-Lys (SEQ ID NO: 8)
Computer searches did not reveal any significant homologies with sequences held in various databases.

### 3) Ion-exchange chromatography.

When in 10 mM Tris-HCl pH 7.4 containing 0.1% (v/v) Triton® X-100, H-gal-GP complex binds to MonoQ ion exchange medium which has been equilibrated with the same buffer. Most of the H-gal-GP complex was eluted from the column between 140 and 400 mM NaCl (Fig. 5 a and b). There was no evidence of separation of the subcomponent bands within this range of salt even if it was applied in small incremental steps.

### 4) Lectin binding

### a) under native conditions

H-gal-GP complex can be isolated by affinity chromatography from a Triton® X-100 extract of Haemonchus membranes using various lectins cross-linked to a solid phase such as agarose beads (Figs. 10 and 11 and Example 2).

The estimated relative affinity of native H-gal-GP complex for certain lectins is summarised in Table 2. With the exception of Dolichos biflorus, lectins with a specificity for N-acetylgalactosamine retained H-gal-GP selectively. The negative result with Dolichos suggested that this affinity was specific for β rather than α linked N-acetylgalactosamines.

The finding that ConA, pea and Lotus lectins also retained H-gal-GP complex indicated that it included glycoproteins bearing mannose and/or fucose, whereas the negative results with Datura and Bandeiraea suggested a lack of N-acetylglucosamine containing structures (Table 2). However, all the lectins which did retain H-gal-GP bound all of it and did not display any differential affinity for its sub-components, as exemplified in Fig. 6.

### b) under dissociated and reduced conditions

In order to determine which sub-components of H-gal-GP were glycosylated, the complex was reduced and dissociated by SDS PAGE, blotted and probed with a panel of biotinylated lectins. The results (Fig 7) showed that some lectins (e.g. lentil) bound to most of the bands whereas others (e.g. peanut, helix pomatia and succinyl wheatgerm) were more selective. All except soybean strongly revealed the 130 kd band and a slightly smaller component, giving the impression of a doublet in this region; some, especially lentil, conA and wheatgerm, stained a diffuse area in the region of the 90kd band; others, particularly wheatgerm, peanut, jacalin, succinyl wheatgerm and helix pomatia, reacted diffusely with a glycoprotein of about 60 kd which stained weakly with Coomassie blue; conA, jacalin, wheatgerm and lentil also bound the 50kd band quite strongly, whereas the 47kd band was only revealed by lentil.

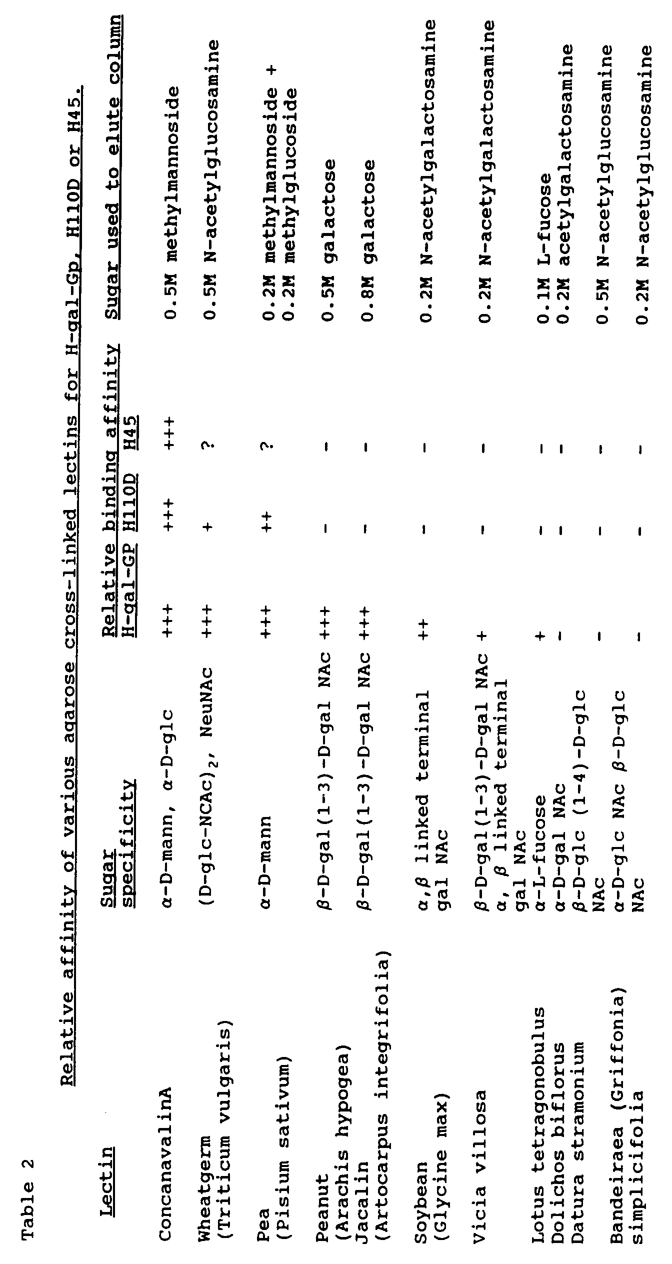

### 5) Protease activity.

H-gal-GP complex shows protease activity with respective pH optima of 4.0 or 6.0 when azocasein or haemoglobin are used as substrates (Figs 7 and 8). This activity is inhibited by pepstatin which binds strongly to H-gal-GP. This last property can be used to enrich for H-gal-GP complex by affinity chromatography.

### 6) Isoelectric point

H-gal-GP complex was subjected to isoelectric focusing within a linear preformed pH gradient (range 2 to 9 pH units) in a Miniphor preparative free-flow apparatus. Most of it eluted between pH 2 and 7.3. This wide range is typical of glycoproteins where variable carbohydrate residues introduce a heterogeneity of charge to the molecules.

### Two methods for preparing H-gal-GP complex from H. contortus

These are summarised in the flow chart in Fig. 4 and in greater detail in Examples 2 and 3.

A detergent extract of Haemonchus membranes (S3) was prepared essentially as described by Munn and Smith (W090/11086).

### EXAMPLE 2

Adult Haemonchus contortus were homogenised at 4°C in acetate buffer or phosphate buffered saline, under acid or neutral conditions containing 1 mM EDTA and 1 mM PMSF as protease inhibitors. The homogenate was centrifuged at 10,000g for 20 minutes under standard conditions and the pellet so formed was extracted with 0.1% Tween® 20 and spun again. This procedure was repeated and the pellet so formed was then extracted with 2% Triton® X-100 and centrifuged under standard conditions. The supernatant was subjected to ultracentrifugation at 100,000g for 1 hour, and the supernatant (S3) was passed through a 0.22 µm filter and diluted fourfold with buffer, namely 10mM Tris - HCl containing 0.5M NaCl, 0.01% NaN₃, pH 7.4.

The diluted supernatant S3 so formed was subjected to lectin affinity chromatography using various lectins bound to agarose beads. After application of the diluted supernatant to the column, washing was effected with several column volumes of buffer and the lectin bound material was then eluted with an appropriate sugar.

The relative amount of H-gal-GP binding to each of 11 different lectins was assessed visually on SDS-PAGE gels as exemplified in Fig. 6. The results in Table 2 showed that whereas several lectins bound to H-gal-GP, only those with a specificity for β linked N-acetylgalactosamine did so specifically and of these Peanut and Jacalin did so most effectively. There was no evidence of separation of the subcomponents of H-gal-GP with any lectin (Fig. 6).

Using peanut or jacalin lectin, the proteins H11OD and H45, together with other material, were washed through, while the H-gal-GP complex remained bound. It was eluted in essentially pure form by 0.3 or 0.8M galactose from peanut and jacalin columns respectively (Fig. 10 and 11).

Using wheat germ lectin a similar result was obtained following elution with 0.5M N-acetylglucosamine except that some H110D was bound and eluted with the H-gal-GP complex (this was the material shown in Track 1 Fig. 11 applied to the jacalin column).

### EXAMPLE 3

### Evidence that pepstatin is a specific and avid ligand for H-Gal-GP

The supernatant S3 in low pH, high ionic strength buffer, namely 20mM sodium acetate, 1M NaCl, pH 5.5, was passed through a column comprising pepstatin bound to agarose beads. Most of the polypeptides, including H110D, did not bind to this matrix (Tracks 1 & 2 Fig. 12). After washing with with several column volumes of this acid buffer, small amounts of H-gal-GP and other loosely bound material were eluted by increasing the pH to 8.5. If the column was then eluted with a buffer solution containing a low concentration of a highly charged detergent (e.g. 1% sodium dodecyl sulphate-SDS), the profile of the polypeptides eluted was very similar to H-gal-GP, although the pair of bands usually observed at about 45 kd on 10% non-reducing gels were diminished (track 4, Fig. 12). If the column was unpacked and aliquots of the agarose were incubated with 5% SDS in the presence of dithiothreitol (DTT), a fraction enriched for these missing bands was eluted, suggesting retention of these was very avid (Track 4, Fig. 13). No protease activity could be detected in the eluted material indicating that the enzyme was not functional in the presence of SDS.

Control experiments were conducted whereby the agarose was removed from the column, after the acid wash stage described above, and washed by repeated centrifugation and resuspension in either buffer alone or buffer containing up to 2% deoxycholate or up to 0.1% SDS. None of these treatments removed significant quantities of H-gal-GP from the agarose indicating that the phenomenon was not merely due to physical entrappment of insoluble protein on the top of the column. The finding that H-gal-GP did not bind to certain lectin-agarose columns (Table 2, Track 5, Fig. 6) provided additional evidence that binding to pepstatin-agarose was ligand specific.

### EXAMPLE 4

### Evidence that H-gal-GP is located in the brush border surface of Haemonchus contortus intestinal cells where it is an integral membrane glycoprotein

### (a) Gut brush border location

i) Cryostat sections of adult H.contortus were stained with a panel of fluorescent-labelled lectins. It was found that both peanut and wheat germ lectin selectively stained the intestinal brush border, (Figs. 14 and 15). Some lectins (e.g. Con A) stained most structures while others (e.g. Ulex) stained little or nothing. This result shows that the brush border of the intestinal cells contains a much higher density of wheat germ and peanut lectin binding sites than other anatomical structures of Haemonchus. Since H-gal-GP binds to peanut and wheat germ lectins it is likely that this glycoprotein is present on the brush border of the intestinal cells.
ii) Cryostat sections of adult H.contortus were incubated with sera from sheep which had been immunised with H-gal-GP in Freunds complete adjuvant or from control sheep which had been injected with adjuvant only (see Example 7, Experiments 3 and 4 for details). After thorough washing the sections were incubated with fluorescein isothiocyanate-conjugated horse antibodies specific for sheep immunoglobulin. After further washing the sections were viewed by fluorescent microscopy.
   The sections incubated with anti H-gal-HP sera showed specific fluorescence of a subcuticular layer as well as the intestinal brush border membrane (Fig. 16). No specific staining was observed on the sections which had been stained with sera from control sheep. These results indicate that H-gal-GP is located on the brush border membrane and in the subcutis of Haemonchus.
iii) Cryostat sections were prepared from adult Haemonchus which had been recovered from sheep which had either been immunised with H-gal-GP in Freunds complete adjuvant or had been injected with adjuvant only (see Example 7, experiments 3 and 4).

The sections were incubated with fluorescein isothiocyanate-conjugated antibodies specific for sheep immunoglobulin. After thorough washing they were viewed under a fluorescent microscope as described above.

Some, though not all, sections of the worms which had survived immunisation with H-gal-GP showed specific fluorescence. This was confined to the brush border of the intestinal cells (Fig. 17). No staining was observed on the Haemonchus sections from control sheep.

This result showed that some worms which survived immunisation with H-gal-GP had sheep immunoglobulin coating their intestinal cell brush border membranes. H-gal-GP must therefore be located on this membrane. The subcuticular staining observed in the 'in vitro' experiment described in the previous section was not seen. Presumably because 'in vivo' host immunoglobulin does not come into contact with this part of the parasite anatomy.

### b) Integral membrane glycoprotein

When periodate treated blots containing PBS, Tween® and Triton® X-100 extracts of whole worms (i.e. S1, S2 and S3 respectively, as described in Fig. 4) were probed with anti-H-gal-GP serum raised in Experiment 3, H-gal-GP was only detected in the Triton® X-100 extract (Fig. 18). This result, by a relatively sensitive technique, inferred that H-gal-GP was not present either as a soluble cytoplasmic protein or as a peripheral membrane protein, rather it required the action of Triton X-100 to liberate it from cell membranes.

This result, together with the lectin binding properties summarised in Table 2, provides ample evidence that H-gal-GP is an integral membrane glycoprotein.

### EXAMPLE 5

### Evidence that H-gal-GP complex is different from either the protein doublet H110D (W090 11086), the protein complex H45 (WO90/11086), the 45 kd membrane protein (W092/13889) or the fibrinolytic protease (EP-A-434909)

### a) Differences between H-gal-GP complex. H110D and the protein complex H45

i) Lectin binding
   The estimated relative affinity of H-gal-GP complex, H110D and the H45 complex for certain lectins is summarised in Table 2. With the exception of Dolichos biflorus which bound to none of them, lectins with a specificity for N-acetylgalactosamine retained H-gal-GP complex but not H110D or H45 complex.
ii) Isoelectric point
   H-gal-GP complex has a lower isoelectric point than either H110D or the H45 complex and can be separated from them by ion exchange chromatography. For example, very little of the H45 complex bound to Mono Q equilibrated with 10 mM Tris-HCl pH 7.4 containing 0.1% (v/v) Triton® X-100, confirming the results of Smith, Munn, Graham, Tavernor and Greenwood (International Journal for Parasitology 1993 23 271-280). The small part that was retained could be eluted before H110D with 50mM NaCl. All H110D was desorbed by 200mM NaCl, that is before most of the H-gal-GP complex started to elute.
iii) SDS-PAGE profile
   The H110D doublet, which remained unchanged at 110 kd when run reduced or non-reduced (Fig. 1 tracks 1 and 4), was obviously distinct from H-gal-GP complex, in both situations (Fig. 1, tracks 1 vs 3 and tracks 4 vs 6). However, because reduction considerably changed the profile of the H45 complex (Fig. 1, tracks 2 vs 5), the difference between it and H-gal-GP complex was more pronounced when the samples were compared unreduced (Fig. 1, tracks 2 vs 3) rather than reduced (Fig. 1, tracks 5 vs 6).
iv) Pepstatin binding
   Neither H110D nor H45 bind to pepstatin agarose, a medium to which H-gal-GP complex avidly adheres (Figs. 12 and 13).

### b) Differences between H-gal-GP complex and the 45kd membrane protein of WO92/13889

i) Lectin binding
   Under native conditions H-gal-GP complex binds to wheatgerm lectin (Table 2, Fig. 19). The protective antigens pursued in W092/13889 did not bind to this lectin, a property specifically employed by the inventors to purify the 45kd membrane protein.
ii) N-terminal amino acid sequences
   The N-terminal amino acid sequence published for the 45kd membrane protein in W092/13889 did not resemble the equivalent data for either the 45,47,48 or 50 kd components of H-gal-GP complex described in Example 1, part 2.
iii) Protective capacity of the denatured antigen
   H-gal-GP complex remained protective for sheep even when denatured following dissociation with SDS and reduction with dithiothreitol (see Example 7, experiments 4 and 5). The 45kd membrane protein described in W092/13889 lost its protective antigen capacity when tested in guinea pigs in denatured form.

### c) Differences between H-gal-GP complex and the fibrinolytic protease

i) Substrate specificity
   Cox, Pratt, Hageman and Boisvenue (Molecular and Biochemical Parasitology 1990 **41**, 25-34) described a fibrinolytic protease from Haemonchus, which they claimed had protective antigen potential despite unconvincing data from 2 small sheep trials (Cox, G.N., Milhausen M. and Hageman R. 1990 European Patent Application No. 434909). H-gal-GP complex did not show any proteolytic activity against fibrinogen (see Table 4 and Example 6 v).
ii) Solubility
   The fibrinolytic protease described by Cox et al 1990 was obtained from a saline extract of Haemonchus. In contrast H-gal-GP complex was not detected in saline extracts and could only be extracted from the parasite with a detergent such as Triton® X-100 (Fig 4.5).

### EXAMPLE 6

### Evidence that H-gal-GP has aspartyl protease properties

i) pH optima
In preliminary tests H-gal-GP purified by peanut lectin was found to hydrolyse azocasein in preference to azocollagen. The optimum pH for this activity was determined over the pH range 4 to 9 using overlapping 0.1M acetate, phosphate or Tris buffers (Fig. 8). Optimal activity was found between pH 4.5 and 6.5.
ii) Inhibitor sensitivity
The inhibitor sensitivity of the proteinase activity was tested with 4 separate preparations of peanut lectin purified H-gal-GP. Enzyme activity was almost completely inhibited by pepstatin in all preparations tested, indicating that proteolysis was due to an aspartyl protease (Table 3).

**TABLE 3**

| Inhibitor sensitivity of protease activity associated with H-gal-GP and the equivalent enzymes from O.circumcincta and O.ostertagi | | | | |
|---|---|---|---|---|
| Inhibitor | Proteinase class indicated | percent activity remaining in the presence of inhibitor | | |
| | | H-gal-GP | Oc-gal-GP | Oo-gal-GP |
| PMSF | serine (thiol) | 100 | 104 | 84 |
| E64 | thiol | 87.8 | 101 | 101 |
| 1, 10 Phenanthroline | metallo | 97 | 94 | 99 |
| EDTA | metallo | 97.8 | ND | ND |
| Pepstatin | carboxyl (aspartyl) | 8.5 | 23 | 0 |
| (Results are the means of 4 separate observations with H-gal-GP and 2 observations with each Ostertagia species ND = not determined). | | | | |

iii) Affinity chromatography on pepstatin agarose
H-gal-GP (purified as described in Example 1 section 1) was passed through a column of pepstatin agarose equilibrated with 25 mM Sodium Acetate buffer, pH 4.5 containing 1M NaCl and 0.1% v/v reduced Triton® X-100 (Do et al 1987, J. Biol. Chem. 262 1037-1043). Extensive washing over the pH range 4.5 to 10 desorbed little protein. However, elution with 1% SDS desorbed most of the H-gal-GP (Fig 20). H-gal-GP did not bind to agarose coupled to a "negative control" ligand (e.g. Dolichos lectin). This result, together with those previously described in Example 3, indicates that H-gal-GP has a strong specificity for pepstatin, classically a characteristic of aspartyl proteases and to a lesser extent of membrane metalloendopeptidases (Zoller, H 1990, Handbook of enzyme inhibitors, published by VCH, Weinheim, Germany). The profile of H-gal-GP on 5 or 10% non-reducing gels was the same before or after binding to pepstatin agarose (Fig. 20). This result suggests that H-gal-GP is either a multimeric enzyme complex, a mixture of pepstatin-binding proteases or a mixture of these enzymes and their precursors.
iv) Substrate gel analysis
H-gal-GP was preincubated in the presence or absence of pepstatin and separated on 7.5% SDS-PAGE gels containing 0.1% w/v gelatin under non-reducing conditions. Following electrophoresis, SDS was eluted by washing in excess Triton® X-100 and the gels were incubated overnight at 37°C in phosphate buffer, pH 6.0. Zones of proteolysis were visualised by Coomassie Blue counterstaining.
Results have been variable but on one occasion proteolysis was correlated to both the major high molecular weight bands of the H-gal-GP complex and the minor components at approximately 45kd. All zones of activity were sensitive to pepstatin. On another occasion activity appeared to be localised to only the 170kd band of the complex and was clearly inhibited by pepstatin (Fig. 21). This variable visualisation of aspartyl proteinases has been described previously and is thought to be due to a relatively poor affinity for gelatin as substrate (Simkin, K.G., Chapman, C.R. and Coles, G.C. 1980. Experimental Parasitology 49, 281-387). Consequently it is likely that H-gal-GP has aspartyl proteinase activity associated with all the bands of the complex observed under non-reducing conditions.
v) Affinity of H-gal-GP for natural blood proteins
H-gal-GP was incubated with fibrinogen, albumin and haemoglobin as well as with azocasein, (all 1 mg/ml in buffer, pH 5.5) prior to precipitation of undigested protein by addition of an equal volume of 1M perchloric acid. Proteolysis was monitored by detection of liberated free alpha-amino nitrogen using the ninhydrin assay (Mathews, B.E. 1977. Symposium of British Society of Parasitology 15: 103-119).
The results show that H-gal-GP has distinct specificity for haemoglobin (Table 4) and that the optimum pH for this activity was 4.0 (Fig. 9).

**TABLE 4**

| Affinity of H-gal-GP for natural blood proteins and azocasein | |
|---|---|
| Substrate | Proteolysis (Absorbance at 563 nm) |
| Fibrinogen | 0.00 |
| Haemoglobin | 5.12 |
| Albumin | 0.77 |
| Azocasein | 0.55 |

vi) Inhibition of H-gal-GP proteolytic activity by serum from immune sheep

Serum from lambs immunised either with H-gal-GP or with adjuvant as described in Example 7, Experiment 3 were incubated with equal aliquots of H-gal-GP. The mixtures were subsequently assayed for proteolytic activity using azocasein as substrate. The results (Table 5) suggest that immunisation with H-gal-GP, elicits a specific inhibiting response, probably due to antibody, against the proteolytic activity of H-gal-GP.

**TABLE 5**

| Immune serum inhibition of H-gal-GP | | |
|---|---|---|
| Serum | % Inhibition of Proteolysis | |
| | Assay 1 | Assay 2 |
| Control lamb | 0 | 2 |
| Immune lamb A | 58 | 55 |
| Immune lamb B | 51 | 59 |

### EXAMPLE 7

### Evidence that sheep immunised with H-gal-GP are protected against challenge with Haemonchus contortus

A series of protection trials were conducted with various fractions of H.contortus. A flow diagram summarising the scheme used to prepare the immunogens for each group of sheep in the five experiments is shown in Figure 22.

### Design of protection experiments

Five immunisation challenge trials involving 19 groups of sheep were conducted in the chronological order outlined below. Within each experiment the groups were balanced for sex and weight.

The planned size of each group was 6 or 7, however one sheep in each of Groups 9 and 10 died before challenge from urinary calculi. The antigens used to immunise each group are shown in Table 6 and Fig 22. Each sheep was immunised on 3 occasions with an equal dose of protein or, in the case of all controls, with adjuvant plus saline only and all were challenged with 5,000 H.contortus larvae. Freunds Complete Adjuvant was used throughout and every inoculum was administered intramuscularily as a 1 or 2ml dose into each back leg.

The age of lamb and timing of events varied slightly between experiments as detailed in Table 7.

### Experiment 1

The objective was to assess the protective capacity of the wheatgerm lectin binding fraction of a Triton® X-100 extract of whole worm membranes.

This immunogen was highly effective at suppressing the faecal egg counts observed in challenge control sheep (Fig 23). Similarily, the immunised group contained significantly fewer worms than the controls at slaughter with a significantly higher than normal male to female ratio (Table 6).

This result contrasted with that described in an experiment in W092/13889 where the wheatgerm lectin binding fraction was poorly protective for guinea pigs. This difference may either reflect differences between the type of challenge system employed or the fact that H110D, the bulk of which does not bind to wheatgerm (Fig. 19), was in fact the principal protective ingredient in the W092/13889 experiment.

The finding that female Haemonchus were more susceptible than males to the effects of immunisation has been reported before in other gut membrane antigen experiments (e.g. Smith, 1993 Res Vet Sci 54 94-101) and is thought to be due to the fact that, being larger, female worms ingest blood at a faster rate than males, therefore receiving noxious antibody at a higher dose rate. Alternatively, this finding may be due to the fact that the anabolic demands of females, because of egg laying, make them more vulnerable to blockage of the nutrient harvesting enzymes. Measuring the sex ratio of recovered worms provides a useful additional parameter for detecting effects caused by successful immunisation by this method.

### Experiment 2

Since it was known that the wheatgerm binding fraction used in Group 1 contained some H110D, a protein already shown to be highly protective for Haemonchus (Munn and Smith 1990), the main objective was to test a wheatgerm binding fraction which had been depleted of H110D by ion-exchange chromatography. To check for the possibilty of non-glycosylated protective membrane proteins, a second group was immunised with a fraction which did not bind to either wheatgerm or ConA lectins (Fig. 22).

Both immunised groups had consistently lower mean egg counts than the controls (Fig. 24), but only in the case of the wheatgerm group was there a significant difference on most sampling days. Neither immunised group contained significantly fewer worms than the controls, although in the wheatgerm group the burden was composed of significantly more male than female worms (Table 6).

Group mean egg and worm counts of the control sheep were substantially lower in this experiment compared to control values in the other 4 trials (Figs. 24 vs 23, 25, and 26 and Table 6), with two animals having very low numbers. This large variation (which probably reflected the use of older sheep) made it difficult to assess the significance, if any, of the protection conferred on Group 3, a situation which demanded a repeat experiment.

### Experiment 3

The objective therefore was to retest the efficacy of the H110D-free wheatgerm binding fraction in younger, more uniformly susceptible lambs. Meanwhile it was found that the same fraction could be produced more simply by affinity chromatography with peanut lectin (Fig. 22) as this ligand did not bind any detectable H110D. Therefore the protective capacities of the peanut binding fraction (i.e. H-gal-GP) and H110D were compared in this experiment.

The faecal egg counts of both groups of immunised lambs were greatly depressed compared to controls (Fig. 25) and significantly fewer worms with an abnormally high male to female sex ratio were recovered from them (Table 6).

It was concluded that H-gal-GP was a protective antigen for Haemonchus.

### Experiment 4

The objective was to confirm the protection result obtained with native H-gal-GP and to determine whether the complex would remain protective when either in a dissociated or in a reduced and dissociated state.

Accordingly an aliquot of H-gal-GP was dissociated by incubation with 0.5% SDS (2.5: 1 w/w SDS : protein) at 25°C for 1 hour. A second identical aliquot was denatured by dissociation with the same amount of SDS combined with 2.5 mM dithiothreitol (DTT) as a reducing agent at 100°C for 5 minutes. Groups 10 and 11 were immunised with H-gal-GP treated in these respective ways whereas Group 9 received an identical but untreated aliquot of native H-gal-GP (Table 6).

Highly significant reductions in both faecal egg output and worm counts were observed between all three immunised groups (Fig 26; Table 6) and the controls, although this effect was most marked in the native and SDS alone groups. As before, female worms were more susceptible than males to the effects of immunisation. It was concluded that the protective epitopes of H-gal-GP were largely unaffected either by dissociation of the complex with SDS or by denaturation when dissociated with SDS and reduced by DTT. This result contrasted with those obtained with the 45 kd membrane glycoprotein in W092/13889 and suggested that the sub-unit polypeptides of H-gal-GP could be tested individually following their separation on and extraction from SDS acrylamide gels.

### Experiment 5

The objective therefore was to test the protective antigen potential of various polypeptide subcomponents of H-gal-GP which had been separated by non-reducing SDS-PAGE.

Groups 13 and 14 were respectively immunised with 500 and 200 µg of H-gal-GP complex which had been dissociated with SDS exactly as described above in Experiment 4. Groups 15 to 18 were immunised with individual components of H-gal-GP excised from 5% gels after the complex had been separated by SDS-PAGE under non-reducing conditions (Fig. 22). As shown in Fig. 2, Group 15 was injected with the 230kd band, Group 16 with the diffuse 190-205kd region, Group 17 with the 170kd band and Group 18 the dyefront bands (Fig. 2). Gel slices containing the appropriate bands were resuspended in a suitable volume of diluent and homogenised with an equal volume of adjuvant. The amount of H-gal-GP fractionated was calculated so that Groups 15 to 18 received a dose of their respective polypeptide equivalent to that present in 500 µg H-gal-GP (ie equivalent to Group 13). A challenge control group (Group 19) was immunised with adjuvant only.

With the exception of one animal (sheep 4), both positive control groups immunised with SDS dissociated H-gal-GP were protected against the challenge infection. This was manifested by reduced egg counts and worm numbers compared to the controls as well as an increase in the proportion of male worms. If sheep 4 is excluded from the calculations the protection figures for Group 13 were:- worms 79% and eggs 87% (counts averaged over days 18-35). For Group 14 the equivalent results were worms 70% and eggs 84%. It was concluded that the reduced dose used for Group 14 did not make any difference and that the result for Group 13 was similar to that obtained in Experiment 4 above.

Much greater individual variation was found in the groups immunised with the various sub-unit bands of H-gal-GP. However the results showed that protection could be obtained with individual sub-unit bands excised from the gels. Of these, Groups 16 (190-205kd diffuse) and 18 (dyefront) were best protected.

Protection in the best subunit groups appeared to be less consistent than in those immunised with the whole complex. This may have been because the process of electrophoretic separation somehow damaged protective epitopes or because a combination of more than one subunit is neccessary for a consistent effect.

It was concluded from these 5 experiments that H-gal-GP was a novel and consistently effective protective antigen complex for Haemonchus whether in its native state or whether dissociated or even reduced and dissociated.

**Table 6**

| Sheep protection experiments 1-4: group mean worm counts and sex ratios of the Haemonchus recovered. | | | | | | |
|---|---|---|---|---|---|---|
| Expt no | Group | n | Antigen | Dose* | Mean count±SE | % Male |
| 1 | 1 | 7 | Wheat germ binding | 200 | 1086±326^{a} | 62.3±1.9^{a} |
| | 2 | 7 | None (control) | - | 3068±416^{b} | 48.6±1.5^{b} |
| 2 | 3 | 6 | Wheat germ minus H110D | 140 | 1809±115^{a} | 63.1±2.8^{a} |
| | 4 | 7 | Lectin unbound | 140 | 1387±350^{a} | 49.6±4.1^{b} |
| | 5 | 7 | None (control) | - | 1644±344^{a} | 51.5±1.3^{b} |
| 3 | 6 | 7 | H110D | 100 | 518±133^{a} | 66.8±5.4^{a} |
| | 7 | 7 | Peanut binding (H-gal-GP) | 200 | 1765±167^{b} | 64.2±3.1^{a} |
| | 8 | 7 | None (control) | - | 3744±187^{c} | 50.1±0.4^{b} |
| 4 | 9 | 5 | Native H-gal-GP | 200 | 1052±424^{a} | 75.5±6.2^{a} |
| | 10 | 5 | H-gal-GP + SDS | 500 | 902±295^{a} | 65.7±3.2^{a} |
| | 11 | 6 | H-gal-GP + SDS + DTT | 500 | 1466±476^{a} | 70.6±5.4^{a} |
| | 12 | 6 | None (control) | - | 3705±627^{b} | 56.1±5.4^{b} |

| | | | | | | |
|---|---|---|---|---|---|---|
| * µg protein injected per sheep per imunisation. Within each experiment values within columns with different superscripts are significantly different. | | | | | | |
| ^{a} age in months when first injected; | | | | | | |
| ^{b} weeks after first "vaccination"; | | | | | | |
| ^{c} weeks between third "vaccination" and challenge; | | | | | | |

**Table 7**

| Differences in the design details of the sheep experiments | | | | |
|---|---|---|---|---|
| Expt no | Age of lamb^{a} | Booster times^{b} | Interval to challenge^{c} | Kill day^{d} |
| 1 | 2 | 4 and 7 | 1 | 23 |
| 2 | 8 | 3 and 6 | 2 | 28 |
| 3 | 2 | 3 and 6 | 2 | 34 |
| 4 | 5 | 3 and 6 | 2 | 35 |
| 5 | 3 | 3 and 6 | 2 | 34 or 35 |

| | | | | |
|---|---|---|---|---|
| ^{a} age in months when first injected; | | | | |
| ^{b} weeks after first "vaccination"; | | | | |
| ^{c} weeks between third "vaccination" and challenge; | | | | |
| ^{d} days after challenge when sheep were killed. | | | | |

### EXAMPLE 8

### Equivalent enzymes in Ostertagia species

Extraction of Ostertagia circumcincta or Ostertagia ostertagi by the method shown in Fig. 4 followed by affinity chromatography with peanut lectin, yields protein complexes which are broadly similar to H-gal-GP as judged by their SDS-PAGE profiles (Fig. 27). Like H-gal-GP, both extracts showed protease activity against azocasein and ovine haemoglobin with pH optima between 4 and 6.0 (Figs. 8 and 9) and the activity was specifically inhibited by pepstatin (Table 3).

Equal quantities of Oc-gal-GP were added to tubes containing agarose beads coupled to either pepstatin, peanut lectin or dolichos lectin and the tubes were gently agitated for 3 hours at 4°C. The beads were pelleted and the unbound supernates were retained. After 3 washes the beads were boiled in a minimum volume of SDS-PAGE sample buffer and pelleted. The supernates from this step, containing material which had bound to the agarose, together with the unbound supernates were run on non-reducing 5% SDS-PAGE gels, blotted to immobilon membrane and probed with biotinylated peanut lectin.

The result depicted in Figure 28 shows that Oc-gal-GP bound to peanut lectin and to pepstatin agarose specifically because it did not bind to agarose coupled with a negative control ligand (dolichos lectin).

Serum from sheep immunized with H-gal-GP as described in Example 7 Experiments 1 and 2 cross-react with protein epitopes of either Ostertagia complex (Fig. 27).

Taken together, these results suggest that H.contortus, O.circumcincta and O.ostertagi share a common family of antigenically related galactose-containing glycoprotein complexes containing pepstatin-inhibitable protease activities.

More specific results are described in the experiments below.

### Experiment 1

### Evidence that lambs immunised with integral membrane proteins of Ostertagia circumcincta are protected against challenge with this parasite

An experiment was conducted where lambs immunised with a Concanavalin A (ConA) binding fraction of Ostertagia circumcincta integral membrane proteins were challenged with infective larvae of this parasite and the degree of protection afforded was measured in relation to a challenge control group.

All antigen preparation procedures were carried out on ice or at 4°C with centrifugation steps at 10,000g for 15 minutes unless otherwise stated. Frozen adult O. circumcincta were thawed and homogenised in buffer (phosphate buffered saline, pH 7.4 containing the following protease inhibitors:- 1mM EDTA, 1mM PMSF and 1mM N-ethylmaleimide). The homogenate was spun and the pellet was resuspended in homogenising buffer containing 0.1% (v/v) Tween® 20. This step was repeated once more and the washed pellet containing worm membranes was resuspended and then extracted by thorough mixing for 2 hours in homogenising buffer containing 2% Triton® X-100 but without EDTA. The extract was spun again and the resulting supernate was ultracentrifuged at 100,000g for 1 hour. This supernate, which is known as S3 (Fig. 29 track 1) and contains solubilised integral membrane proteins, was passed through a 0.22 µm pore filter, diluted 4-fold with buffer (10mM Tris-HCl pH 7.4, containing 0.5M NaCl and 0.01% w/v NaN₃) and applied to a ConA-agarose column. Unbound material (Fig. 29, tracks 3 and 4) was discarded and, after thorough washing, the bound fraction was eluted with 200mM α-methyl mannoside/α-methyl glucoside (Fig. 29, track 2). This fraction was desugared and desalted by gel filtration (Sephadex® G-25, Pharmacia), concentrated by ion exchange on Mono Q (Pharmacia) and diluted in cold phosphate buffered saline (PBS) prior to emulsification with an equal volume of Freunds complete adjuvant.

Fourteen Suffolk cross lambs, which had been reared and housed in conditions designed to exclude nematode parasites, were allocated to 2 groups each balanced for sex and weight and consisting of 6 immunised and 8 control lambs. At about 7 months of age the immunised group were each injected with 100 µg antigen and the control lambs were injected with adjuvant only. One ml of vaccine was injected into each hind leg in the semimembranous and semitendinosus muscles. Each group received further identical inoculations 3 and 6 weeks later. Two weeks after the final boost all lambs were challenged orally with 5,000 third stage O. circumcincta larvae. Faecal egg counts were made twice weekly from 2 weeks post challenge and the animals were killed for total worm counts 5 weeks after challenge.

The group mean faecal egg counts of the immunised lambs were significantly lower than those of the control group throughout the experiment (Table 8 and Fig. 30). A comparison of the group means averaged between days 21 and 35 of the experiment, revealed that the egg output of the immunised group was reduced by more than 80%.

Subsamples (5-10%) of the abomasal contents and mucosal digests were searched for worms. No arrested early fourth stage larvae were found, all worms having developed as far as the fifth or adult stages. Comparison of the group worm burdens showed that they were about 60% lower in the immunised sheep, a statistically significant difference (Table 8).

**Table 8**

| Faecal egg counts and total Ostertagia burdens in immunised and control sheep | | | | | | | | | |
|---|---|---|---|---|---|---|---|---|---|
| Group | Sheep | Days after challenge infection | | | | | | | Worms^{a} |
| | | 14 | 18 | 21 | 25 | 28 | 32 | 35 | |
| Immunised | 1 | 0^{b} | 45 | 198 | 30 | 108 | 30 | 162 | 2673 |
| | 2 | 0 | 0 | 1 | 8 | 36 | 24 | 15 | 321 |
| | 3 | 0 | 0 | 1 | 12 | 27 | 15 | 48 | 905 |
| | 4 | 0 | 0 | 2 | 24 | 33 | 15 | 36 | 322 |
| | 5 | 7 | 207 | 315 | 162 | 108 | 63 | 207 | 2563 |
| | 6 | 0 | 1 | 63 | 72 | 198 | 135 | 189 | 2914 |
| | **mean** | **1.17** | **42.2** | **96.7** | **51.3** | **85.0** | **47.0** | **109.5** | **1616** |
| Control | 7 | 0 | 342 | 459 | 72 | 495 | 387 | 450 | 3830 |
| | 8 | 6 | 198 | 450 | 297 | 720 | 432 | 558 | 4979 |
| | 9 | 13 | 261 | 585 | 297 | 603 | 927 | 603 | 3292 |
| | 10 | 11 | 432 | 441 | 252 | 252 | 693 | 612 | 3993 |
| | 11 | 7 | 279 | 405 | 207 | 378 | 486 | * | 3806 |
| | 12 | 9 | 396 | 297 | 369 | 1134 | 495 | 459 | 4189 |
| | 13 | 3 | 27 | 189 | 108 | 72 | 162 | 198 | 2088 |
| | 14 | 27 | 333 | 486 | 450 | 288 | 531 | 639 | 4568 |
| | **mean** | **9.62** | **283.5** | **414.0** | **256.5** | **493.0** | **514.1** | **502.7** | **3843** |
| | P value | <0.05 | <0.01 | <0.01 | <0.01 | <0.02 | <0.01 | <0.01 | <0.01 |

| | | | | | | | | | |
|---|---|---|---|---|---|---|---|---|---|
| a - total worm count | | | | | | | | | |
| b - no. of eggs/g of faeces | | | | | | | | | |
| * - no sample P value - significance of difference between the respective means of the immunised and control groups by the T test. | | | | | | | | | |

### Experiment 2

### Evidence that the protective antigens include the Ostertagia circumcincta galactose containing glycoprotein complex (Oc-gal-GP)

a) The ConA binding fraction used to immunise the sheep in Experiment 1 was electrophoresed on 10% reducing SDS-PAGE gels and blotted onto immobilon membrane. The blots were probed with biotinylated peanut lectin, the ligand shown to be specific for Oc-gal-GP (Figs. 28 + 29). The positive result (Fig. 31) showed that Oc-gal-GP was present in the protective antigen preparation.
b) To prepare Oc-gal-GP, adult O.circumcincta were processed as far as the S3 stage which was pumped through a column containing peanut lectin-agarose as described in Experiment 1 above. The column was washed with several column volumes of buffer and then the bound material was eluted with 0.5M galactose to obtain Oc-gal-GP.
   Blots of Oc-gal-GP were prepared as described above, except that they were first treated with periodate to disrupt carbohydrate epitopes and reduce potential cross-reactions. (Woodward, Young and Bloodgood, 1985, Journal of immunological methods 78:143-153). The blots were probed either with immune serum from Example 8, Experiment 1 or with a gastric lymph sample from sheep naturally immune to O.circumcincta (Smith, Jackson, Jackson, and Williams, 1985, Journal of Comparative Pathology 95:235-275). The anti-serum to the ConA-binding antigen from Experiment 1 reacted with Oc-gal-GP but no reaction was seen with the lymph from naturally immune animals. These results supported the observation in Experiment 2 (a) above that Oc-gal-GP was present in the ConA binding fraction used to immunise the sheep and showed that Oc-gal-GP is also a "hidden" antigen.

### Experiment 3

### Evidence that Oc-gal-GP is a gut membrane protein

Cryostat sections of O.circumcincta were prepared and stained with fluorescein conjugated peanut lectin. Intense fluorescence was observed over the gut although as yet unidentified internal structures also stained less intensely (Fig. 32). Since peanut lectin specifically binds Oc-gal-GP, this result indicates that Oc-gal-GP is a gut membrane protein.

### EXAMPLE 9

### Equivalent enzymes in Trichostrongylus species

Proteolytic activity in extracts of adult T.vitrinus was reduced by 40% in the presence of pepstatin. In addition the activity of a proteolytically active doublet (Mw about 205 Kd), visualised under non-reducing conditions by gelatin-substrate gel analysis, was markedly reduced in the presence of pepstatin. These observations indicate that T.vitrinus also contains aspartyl proteinase activity similar to Haemonchus and Ostertagia spp.

### EXAMPLE 10

### Evidence that H-gal-GP contains a neutral endopeptidase

### 1. Detection of neutral endopeptidase-like activity associated with H-gal-GP

### Methods

Proteinase activity was determined using azocasein as substrate in a microassay system. H-gal-GP (approximately 5ug in 20µl) was preincubated for 3Omin at 37°C with 2OOµl sterile 0.1M phosphate buffer, pH 7.0 containing no inhibitor (control), or inhibitor to a final concentration of 1mM. Inhibitors used were prepared as 100mM stock solutions in water except for 4-hydroxymercuribenzoate (4HMB) and pepstatin which were dissolved in methanol. Inhibitors tested were dithiothreitol (DTT); L-cysteine; reduced glutathione; 4HMB; N-ethylmaleimide; EDTA; 1,10 phenanthroline and pepstatin and were all purchased from Sigma. After preincubation 20µl azocasein (5mg/ml in sterile distilled water; Sigma) was added and the reaction mixtures incubated overnight at 37°C. Parallel blanks containing sterile water instead of H-gal-GP were included for each inhibitor tested and assays were performed in duplicate. The reactions were stopped by addition of an equal volume of 1M perchloric acid (BDH chemicals) and retained on ice for 15min to precipitate undigested protein which was subsequently pelleted by brief centrifugation at 11000g using a microfuge. The 0D₄₀₅ of the supernatant was determined using a "Monarch" microcentrifugal analyser (Instrumentation Laboratory, Warrington, UK). After subtraction of the appropriate reagent blank the degree of inhibition was expressed as the percent activity remaining compared to the control reaction.

### 2. Isolation and characterisation of neutral endopeptidase encoding lambda gtll clones

### 1. Isolation of poly (A)+ mRNA from adult worms

Adult H.contortus were obtained from the abomasa of experimentally infected sheep, washed with saline and snap frozen in liquid nitrogen. The frozen parasites were ground to a fine powder, using a pestle and mortar which had been prechilled to -70°C, and solubilised in 5ml extraction buffer (4M guanidine isothiocyanate, 25mM sodium citrate, 0.5% (w/v) sarcosyl and 0.7% (v/v) 2-mercaptoethanol in distilled water). After the addition of 5ml phenol/chloroform the solution was mixed vigorously by vortexing for 5min and then incubated on ice for 10min. Aqueous and organic phases were separated by centrifugation at 10000g and 4°C for 30min, the aqueous phase retained and RNA precipitated by addition of an equal volume of isopropanol and chilling at -20°C for 1 hour. RNA was pelleted, redissolved in extraction buffer and repelleted as described above. The pellet was washed with 70% ethanol, dried and dissolved in 0.5ml sterile distilled water.

Poly (A)+ mRNA was isolated by oligo-dT-cellulose chromatography using the methods described by Efstratiadis et al (1976, Cell, 7 279-288). The poly (A)+ mRNA was precipitated with ethanol, dried and redissolved in 10 to 2Oµl sterile distilled water.

### 2. cDNA synthesis and λgt11 library preparation

Double-stranded cDNA was prepared from 1µg of poly (A)+ mRNA using a kit purchased from Amersham according to the manufacturers instructions. First strand synthesis was performed using AMV reverse transcriptase and the reaction primed using random hexanucleotide primers. Second strand synthesis was performed using RNase H and E.coli DNA polymerase 1. Finally, T4 DNA polymerase was used to blunt-end the cDNA. The cDNA was ligated into bacteriophage λgtll (λgt11) using an Amersham kit (RPN 1280). Briefly, EcoR1 adaptors were ligated to the cDNA pool, unligated adaptor molecules removed by gel filtration and cDNA >500bp retained for ligation into λgt11 arms. The "adapted" cDNA ends were phosphorylated in a reaction with T4 polynucleotide kinase and different amounts (100, 75 & 40ng) ligated into λgt11 arms (1µg). The ligation mixes were packaged in vitro (Boehringer) and aliquots plated on E.coli Y1090 in the presence of isopropylthio galactopyranoside (IPTG) and 5-bromo-4-chloro-3-indolyl-β-D-galactopyranoside (X-gal) to enable recombinant titre to be assessed. Each ligation reaction yielded approximately 2 X 10⁵ recombinant phage. Phage expressing H-gal-GP components were selected by immunoscreening 10⁵ recombinant phage using sheep anti-H-gal-GP antiserum (see below).

### 3. Immunoscreening

The λgt11 cDNA expression library was plated on a lawn of E.coli Y1090 in 90mm petri dishes at a density of 1000 phage per L-agar/ampicillin plate. The plates were incubated at 42°C until plaques were just visible (4h) and then overlayed with nitrocellulose filters which had been saturated in 10mM IPTG and air dried. The plates with filters were incubated at 37°C overnight. The next day the filters were removed and extensively washed in TBST (50mM Tris-base, 150mM NaCl, pH8.0 containing 0.05% v/v Tween®-20) and then blocked by incubation in 5% horse serum in TBST for 1 hour. The filters were then incubated with sheep anti-H-gal-GP antiserum diluted 1/500 in TBST containing 5% horse serum for 4 hours. After further extensive washing with TBST the filters were incubated with peroxidase conjugated horse anti-sheep immunoglobulin antiserum (1/200 in TBST) for 2 hours and, washed, and immunopositive phage visualised by incubation with DAB development solution (10mg diaminobenzidine-HCl in 50ml H₂0 containing 5Oul 30% hydrogen peroxide, Sigma). Immunopositive plaques were cored out and replated to plaque purity by successive replating/immunoscreening cycles. Screening of 10⁵ phage yielded 7 positive phage. These were further analysed by antibody elution.

### 4. Antibody elution

The recombinant phage were plated at a density of 10³ per 90mm plate on E.coli Y1090 and incubated at 42°C for 4 hours. The plates were overlayed with IPTG saturated nitrocellulose filters and incubated at 37°C overnight. The filters were removed and washed, extensively, with TBST prior to incubation with sheep anti-H-gal-GP serum for 4 hours. Bound antibody was eluted by two applications of elution buffer (5mM glycine, 500mM NaCl, 0.2% Tween®-20, pH2.3) for 2 minutes each. The resulting antibody solution (4ml) was neutralised by the addition of 200ul 1M Tris-HCl, pH 7.4, diluted three-fold with 5% horse serum in TBS (TBST with Tween®-20 omitted) and incubated overnight with Western blot strips of H-gal-GP separated by 7.5% SDS-PAGE. Subsequent washing procedures incubations with second antibody and visualisations using DAB solution were as described above.

### 5. Polymerase chain reaction (PCR) amplification of cDNAs inserts in immunopositive phage and Southern blotting

Inserted cDNA was obtained in preparative amounts by PCR amplification using primers directed to regions flanking the EcoR1 cloning site in λgt11 (Saiki et al, 1988 Science 239 487-491). Briefly, phage particles were eluted from cored out plaques into 5Oµl sterile distilled water in a 0.5ml eppendorf tube. After a brief freeze/thaw cycle 25µl of the supernatant was used in a PCR reaction as described by Saiki et al, 1988. Primers were annealed at 55°C. PCR products were fractionated in either 0.8% agarose gels or in 7.5% continuous polyacrylamide gel slabs and the products visualised by ethidium bromide staining (agarose) or silver staining (polyacrylamide).

In addition, the products of PCR amplifications of cDNA inserts were fractionated in agarose gels and Southern blotted onto nylon membranes (Hybond-N, Amersham) by the capillary transfer technique described in Maniatis et al, (1982), in Molecular Cloning: a laboratory manual Cold Spring Harbour Laboratory. DNA (100ng) for probing the blot was prepared by PCR amplification of phage HG3 as outlined above and labelled by the Digoxygenin methodology according to the manufacturers instructions (Nucleic acid labelling and detection kit, Boehringer). Hybridisation was conducted under conditions of high stringency (5 X SSC, 0.5% w/v blocking reagent, 0.1% w/v N-laurylsarcosine, 0.02% w/v SDS in 50% formamide) at 42°C overnight. Probe DNA was added at a final concentration of 25ng/ml in the hybridisation solution. After high stringency washing (final wash 0.1 X SSC, 0.1% SDS for 15 minutes at 65°C) hybridisation was visualised by immunological detection using alkaline phosphatase conjugated anti-digoxygenin and an enzymically catalysed colour reaction (Boehringer).

### 6. Sequence analysis

PCR amplified cDNA inserts were subcloned into the PCR 1000 vector (In-Vitrogen) as detailed by the manufacturers. DNA sequence was determined by the dideoxy chain termination procedure (Sanger et al, 1977 Proceedings of National Academy of Science 79 5463-5467). Sequencing reactions were performed using the Pharmacia T7 sequencing kit and primer annealing and sequencing reactions were performed precisely as detailed in the kit instructions. The reaction products were fractionated in 6% polyacrylamide, 50% urea gel slabs with Tris/Acetate/EDTA running buffer for up to 6 hours at 5OW constant power. Following electrophoresis, gels were soaked in acetic acid:methanol:water (5:5:90) for 40 minutes, dried onto Whatman 31 filter paper and resolved DNA fragments were then detected by autoradiography at room temperature overnight. The DNA and deduced amino acid sequences were compared to existing sequences in the GENBANK and EMBL databases.

### RESULTS

### 1. Neutral endopeptidase in HgalGP - biochemical detection

Neutral endopeptidase-like activity associated with H-gal-GP was detected by differential proteinase inhibitor sensitivity using azocasein as substrate. The results of a typical analysis are shown in Table 9.

**Table 9**

| The sensitivity of azocasein proteolysis by H-gal-GP at pH7 to a panel of proteinase inhibitors. | | |
|---|---|---|
| Inhibitor | % activity remaining | |
| No inhibitor control | 100 | 100 |
| Dithiothreitol | 48 | 44 |
| Cysteine | O | 0 |
| Glutathione | 87 | 85 |
| 4-hydroxymercuribenzoate | 88 | 91 |
| N-ethylmaleimide | 74 | 77 |
| EDTA | 25 | 10 |
| 1,10 phenanthroline | 26 | 23 |
| pepstatin | 75 | 85 |

% inhibition is expressed as the percent activity remaining in comparison to a no inhibitor control. The results of two separate analyses are shown on the same batch of H-gal-GP.

Enzyme activity was inhibited by the chelating agents EDTA and 1,10 phenanthroline as well as the thiol reagents dithiothreitol and cysteine. Activity was relatively unaffected by 4-hydroxymercuribenzoate, N-ethylmaleimide, pepstatin and glutathione. This inhibition profile is consistent with that reported previously for neutral endopeptidases of mammalian origin (reviewed by Kenny, 1977. In: Proteinases in mammalian cells and tissues, Ed: Barrett, A.J. Elsevier).

### 2. Molecular analysis of H-gal-GP positive λGt11 recombinants

Immunoscreening 10⁵ λgt11 recombinants yielded 7 immunopositive phage. Antibody elution experiments showed that only antibody eluted from recombinant phage designated HG3 clearly recognised H-gal-GP on Western blot strips prepared from reducing SDS-PAGE gels. Antibody eluted from non-recombinant λgt11 did not recognise H-gal-GP components demonstrating the specificity of the HG3 reaction. Amplified insert DNA from HG3 was used to probe a Southern blot of insert DNA amplified from the other immunopositive plaques and recombinant HG11 gave a positive hybridisation signal. The size of the cDNA inserts in HG3 and HG11 was 212bp and 1O19bp respectively.

The nucleotide (Figure 33) and amino acid (Figure 34) sequences of HG3 were determined in full. HG3 showed 33% identity and 65% similarity with human neutral endopeptidase, ie. very significant homologies. Sequence analysis of HG11 is continuing but the region corresponding to HG3 has been identified and shows 96% identity at the nucleotide level. Therefore HG11 appears to be a variant of HG3. Such variation may be due to the presence of natural biological variants between different strains and different life cycle stages of the helminth. Moreover there may exist multiple enzyme forms (isoenzymes) which may be expressed differentially during parasite development or in strains of different origin.

### Example 11

### To show the existence of neutral endopeptidase - encoding genes in various metazoan parasites; hybridisation of clone HG11 to genomic DNA from metazoan parasites digested with EcoRI

In order to prepare genomic DNA from various metazoan parasites (Haemonchus, Lucilia, Nematodirus, and Boophilus) two grammes of each which had been snap-frozen in liquid nitrogen were ground to a fine powder in liquid nitrogen. The powder was added slowly to 25ml of lysis buffer (0.05M Tris-HCL pH8.0, 0.1M EDTA, 1% w/v Sarkosyl, 0.05mg/ml proteinase K) and incubated for two hours at 65°C. The suspension was then extracted twice with one volume of phenol plus chloroform, twice with two volumes of chloroform and then ethanol precipitated. The pellet was resuspended in 20 ml of Tris , EDTA buffer (TE, pH8.0) overnight at 4°C on a rocking table, then dialysed against two changes of one litre of TE. The RNA was removed by incubating with DNase-free RNase A type I (Sigma) at a final concentration of 20ug/ml, for one hour at 37°C, then extracting once with phenolchloroform once with chloroform and then ethanol precipitating, as above. The pellet was washed twice with 70% v/v ethanol and resuspended in one ml of TE as above.

5µg of genomic DNA was digested with EcoRI overnight at 37°C and then electrophoresed at 5ug per track on a 1% w/v agarose gel in Tris-acetate buffer. The gel was Southern blotted by capillary transfer as described in Maniatis et al (1982) onto Hybond-N membrane (Amersham International). DNA was fixed onto the membrane using ultraviolet light, according to the manufacturer's recommendations.

The HG11 clone was digested with SacI and BamHI, this was electrophoresed and a 750bp fragment recovered. The insert was radiolabelled with _ ³²P-dCTP using a Nick Translation kit from Promega Biotech according to the manufacturer's directions. Labelled DNA was separated from unincorporated label by spin column chromatography.

The Southern blot was prehybridised in hybridisation solution (6X SSC, 50% formamide, 5X Denhardt's solution, 0.1% SDS) for 3 hours at 28°C and then hybridised at moderate stringency in the presence of the radiolabelled probe overnight at 28°C. The blot was then washed for 10 minutes at room temperature, followed by a second wash for an hour at 42°C, both were in 2X SSC, 0.1% SDS. Following autoradiography the blot was stripped of probe by incubation for 30 minutes at 42°C in 0.4N sodium hydroxide. The removal of probe was confirmed by exposure to autoradiograph overnight.

The blot was then rehybridised under low stringency conditions using the same probe. Pre-hybridisation and hybridisation were as described above except that a temperature of 24°C was used. Washings were also as described above except that the second wash was performed at 24°C for 30 minutes.

The Southern blot using the HG11 probe showed that not only was there binding to DNA of Haemonchus origin but to that of the other metazoan parasites Haemonchus, Lucilia, Nematodirus, Boophilus as shown in tracks 1 to 4 respectively of Figure 35. This was confirmed by hybridisation under conditions of both moderate and low stringency.

## Claims

1. A protective metazoan parasite antigen or antigenic fragments, components or precursors thereof and functionally-equivalent derivatives, analogues, or variants thereof having protective antigenic activity against one or more metazoan parasites, which antigen is characterised by being capable of binding to pepstatin, and which in native form is an integral membrane protein or protein complex.

2. An antigen, antigenic fragment, component, precursor or functionally-equivalent derivative, analogue or variant thereof, as claimed in claim 1, further characterised by;
(a) possessing proteolytic activity;
(b) in native form being located in the intestinal brush border of the parasite gut; and
(c) being capable of binding to wheatgerm lectin and to lectins having specificity for β-linked N-acetylgalactosamine.

3. An antigen, antigenic fragment, component, or precursor or functionally-equivalent derivative, analogue, or variant thereof as claimed in claim 1, which in native state is capable of binding to Con A, pea, lotus, Helix pomatia, peanut and jacalin lectins.

4. An antigen, antigenic fragment, component or precursor thereof or functionally-equivalent derivative, analogue or variant thereof as claimed in any one of claims 1 to 3 wherein the antigen is further characterised by possessing aspartyl proteinase-like and/or neutral endopeptidase-like activity.

5. An antigen, antigenic fragment, component or precursor or functionally-equivalent derivative, analogue or variant thereof as claimed in any one of claims 1 to 4, obtainable from helminths of the genera Haemonchus, Ostertagia or Trichostrongylus.

6. An antigen, antigenic fragment, component or precursor or functionally-equivalent derivative, analogue or variant thereof as claimed in any one of claims 1 to 5 having the SDS-PAGE molecular weight distribution set out in Table 1, or a component thereof.

7. An antigen, antigenic fragment, component or precursors thereof, or functionally-equivalent derivative, analogue or variant thereof as claimed in any one of claims 1 to 6 which antigen is obtainable from the gut of Haemonchus sp. and has a molecular weight of 190 to 205 kd as determined by SDS-PAGE on a 5% gel under non-reducing conditions or 35 to 50 kd as determined by SDS-PAGE on a 10% gel under non-reducing conditions.

8. An antigen, antigenic fragment, component or precursor or functionally-equivalent derivative, analogue or variant thereof as claimed in any one of claims 1 to 7, for use in stimulating an immune response against metazoan parasites in a human or non-human animal.

9. Use of a metazoan parasite antigen and components, fragments, precursors and functionally-equivalent derivatives, analogues or variants thereof as claimed in any one of claims 1 to 7, for the preparation of a vaccine composition for use in stimulating an immune response against metazoan parasites in a human or non-human animal.

10. A vaccine composition for stimulating an immune response against metazoan parasites in a human or non-human animal, comprising one or more antigens or components, fragments, precursors and functionally equivalent derivatives, analogues or variants thereof as claimed in anyone of claims 1 to 7, together with a pharmaceutically acceptable carrier or diluent.

11. A composition as claimed in claim 10, comprising one or more of the said antigens together with one or more additional antigens selected from the antigens, H11OD and H45 and their components.

12. An antibody, or antigen-binding fragment thereof, which is capable of selectively binding either to an antigen, antigenic fragment, component or precursor or functionally-equivalent derivative, analogue or variant thereof as defined in any one of claims 1 to 7, or to the idiotype of a said antigen-binding antibody.

13. A process for the preparation of a vaccine for use in immunising a human or non-human animal against a metazoan parasite infection, said process comprising preparing an extract of said parasite containing at least one protective antigen as defined in any one of claims 1 to 7, purifying said antigen therefrom by binding said antigen to an immobilized phase including a specific binding partner for the antigen and subsequently eluting said antigen from said immobilized phase.

14. A method for the identification of protective antigens in a metazoan parasite, comprising subjecting an integral membrane fraction of the said parasite to affinity chromatography using an immobilised phase carrying one or more lectins with specificity for N-acetyl-galactosamine.

15. A method as claimed in claim 13 or claim 14 wherein said specific binding partner or lectin is peanut or jacalin lectin.

16. A nucleic acid molecule comprising a nucleotide sequence encoding an antigen, antigenic fragment, component or precursor or functionally-equivalent derivative, analogue or variant thereof as defined in any one of claims 1 to 7.

17. A nucleic acid molecule as claimed in claim 16 which comprises one or more nucleotide sequences substantially corresponding to all or a portion of the nucleotide sequence shown in Figure 33 (SEQ ID NO:
1) or a sequence which is degenerate or substantially homologous with or which hybridises with said sequence.

18. An expression or cloning vector comprising a nucleic acid molecule as defined in claim 16 or claim 17.

19. A host cell or a transgenic organism containing a nucleic acid molecule as defined in claim 16 or claim 17.

20. A method for preparing an antigen, antigenic fragment, component or precursor or functionally-equivalent derivative, analogue or variant thereof as defined in any one of claims 1 to 7, which comprises culturing a host cell containing a nucleic acid molecule encoding all or a portion of said antigen, under conditions whereby said antigen is expressed and recovering said antigen thus produced.

21. A protective metazoan parasite antigen or antigenic fragments, components or precursors thereof and functionally-equivalent derivatives, analogues or variants thereof having protective antigenic activity against one or more metazoan parasites, which antigen is characterised by possessing aspartyl proteinase-like and/or neutral endopeptidase-like activity and which in native form, is an integral membrane protein or protein complex.

22. A protective metazoan parasite antigen or antigenic fragments, components or precursors thereof and functionally-equivalent derivatives, analogues or variants thereof having protective antigenic activity against one or more metazoan parasites, which antigen is, or forms part of, an integral membrane protein or protein complex obtainable from the gut of Haemonchus sp. and has a molecular weight of 190 to 205 kd as determined by SDS-PAGE on a 5% gel under non-reducing conditions or 35 to 50 kd as determined by SDS-PAGE on a 10% gel under non-reducing conditions.

## Patentansprüche

1. Schützendes Metazoen-Parasiten-Antigen oder antigene Fragmente, Komponenten oder Vorstufen davon und funktionell äquivalente Derivate, Analoga oder Varianten davon mit schützender antigener Aktivität gegenüber einem oder mehreren Metazoen-Parasiten, wobei das Antigen dadurch gekennzeichnet ist, daß es zur Bindung an Pepstatin imstande ist und wobei das Antigen in nativer Form ein integrales Membranprotein oder ein Proteinkomplex ist.

2. Antigen, antigene(s) Fragment, Komponente, Vorstufe oder funktionell äquivalente(s) Derivat, Analogon oder Variante davon gemäß Anspruch 1, ferner gekennzeichnet durch
(a) den Besitz proteolytischer Aktivität;
(b) die Anordnung in nativer Form im intestinalen Bürstensaum des Parasitendarms; und
(c) die Fähigkeit zur Bindung an Weizenkeim-Lectin und Lectine mit einer Spezifität für β-verknüpftes N-Acetylgalactosamin.

3. Antigen, antigene(s) Fragment, Komponente oder Vorstufe davon oder funktionell äquivalente(s) Derivat, Analogon oder Variante davon gemäß Anspruch 1, das/die im nativen Zustand zur Bindung an Con A, Erbse-, Lotus-, Helix pomatia-, Erdnuß- und Jacalin-Lectin imstande ist.

4. Antigen, antigene(s) Fragment, Komponente oder Vorstufe davon oder funktionell äquivalente(s) Derivat, Analogon oder Variante davon nach einem der Ansprüche 1 bis 3, wobei das Antigen ferner dadurch gekennzeichnet ist, daß es Aspartylproteinase-ähnliche und/oder Neutralendopeptidase-ähnliche Aktivität besitzt.

5. Antigen, antigene(s) Fragment, Komponente oder Vorstufe oder funktionell äquivalente(s) Derivat, Analogon oder Variante davon gemäß einem der Ansprüche 1 bis 4, das/die aus Helminthen der Gattungen Haemonchus, Ostertagia oder Trichostrongylus erhältlich ist.

6. Antigen, antigene(s) Fragment, Komponente oder Vorstufe oder funktionell äquivalente(s) Derivat, Analogon oder Variante davon nach einem der Ansprüche 1 bis 5 mit einer SDS-PAGE-Molekulargewichtsverteilung, wie sie in Tabelle 1 angegeben ist, oder eine Komponente davon.

7. Antigen, antigene(s) Fragment, Komponente oder Vorstufe davon oder funktionell äquivalente(s) Derivat, Analogon oder Variante davon nach einem der Ansprüche 1 bis 6, wobei das Antigen aus dem Darm von Haemonchus sp erhältlich ist und ein Molekulargewicht von 190 bis 205 kD gemäß einer Bestimmung durch SDS-PAGE auf einem 5 %-igen Gel unter nichtreduzierenden Bedingungen oder von 35 bis 50 kD gemäß einer Bestimmung durch SDS-PAGE auf einem 10 %-igen Gel unter nicht-reduzierenden Bedingungen aufweist.

8. Antigen, antigene(s) Fragment, Komponente oder Vorstufe oder funktionell äquivalente(s) Derivat, Analogon oder Variante davon nach einem der Ansprüche 1 bis 7 zur Verwendung bei der Stimulierung einer Immunreaktion gegen Metazoen-Parasiten in einem Menschen oder einem nichtmenschlichen Tier.

9. Verwendung eines Metazoen-Parasiten-Antigens und von Komponenten, Fragmenten, Vorstufen, funktionell-äquivalenten Derivaten, Analoga oder Varianten davon nach einem der Ansprüche 1 bis 7 für die Herstellung einer Vaccinzusammensetzung für die Verwendung bei der Stimulierung einer Immunreaktion gegen Metazoen-Parasiten in einem Menschen oder einem nicht-menschlichen Tier.

10. Vaccinzusammensetzung zur Stimulierung einer Immunreaktion gegen Metazoen-Parasiten in einem Menschen oder einem nicht-menschlichen Tier, die ein oder mehrere Antigene oder Komponenten Fragmente, Vorstufen und funktionell äquivalente Derivate, Analoga oder Varianten davon nach einem der Ansprüche 1 bis 7 zusammen mit einem pharmazeutisch verträglichen Träger oder Verdünnungsmittel umfaßt.

11. Zusammensetzung nach Anspruch 10, die ein oder mehrere der Antigene zusammen mit einem oder mehreren weiteren Antigenen, die unter den Antigenen H110D und H45 sowie ihren Komponenten ausgewählt sind, umfaßt.

12. Antikörper oder Antigen-bindendes Fragment davon, der/das zur selektiven Bindung an ein Antigen, ein/eine antigenes/antigene Fragment, Komponente oder Vorstufe oder ein/eine funktionell äquivalentes/äquivalente Derivat, Analogon oder Variante davon gemäß der Definition in einem er Ansprüche 1 bis 7 oder an den Idiotypus eines derartigen Antigen-bindenden Antikörpers imstande ist.

13. Verfahren zur Herstellung eines Vaccins zur Verwendung bei der Immunisierung eines Menschen oder eines nicht-menschlichen Tiers gegen eine Metazoen-Parasiten-Infektion, wobei das Verfahren die Herstellung eine Extrakts des Parasiten, der mindestens ein schützendes Antigen gemäß der Definition in einem der Ansprüche 1 bis 7 enthält, die Reinigung des Antigens daraus durch Bindung des Antigens an eine immobilisierte Phase, die einen spezifischen Bindungspartner für das Antigen enthält, und die anschließende Elution des Antigens aus der immobilisierten Phase umfaßt.

14. Verfahren zur Identifizierung schützender Antigene in einem Metazoen-Parasiten, wobei es das Verfahren umfaßt, eine integrale Membranfraktion des Parasiten einer Affinitätschromatographie unter Verwendung einer immobilisierten Phase, die ein oder mehrere Lectine mit einer Spezifität für N-Acetylgalactosamin trägt, zu unterwerfen.

15. Verfahren nach Anspruch 13 oder 14, wobei es sich bei dem spezifischen Bindungspartner oder Lectin um Erdnuß- oder Jacalin-Lectin handelt.

16. Nucleinsäuremolekül, das eine Nucleotidsequenz umfaßt, die für ein Antigen, ein/eine antigenes/antigene Fragment, Komponente oder Vorstufe oder ein/eine funktionell äquivalentes/äquivalente Derivat, Analogon oder Variante davon gemäß der Definition in einem der Ansprüche 1 bis 7 kodiert.

17. Nucleinsäuremolekül nach Anspruch 16, das eine oder mehrere Nucleotidsequenzen umfaßt, die im wesentlichen der gesamten Nucleotidsequenz, die in Fig. 33 (SEQ-ID-Nr.: 1) gezeigt ist, oder einem Teil davon oder einer Sequenz, die degeneriert oder im wesentlichen homolog zu der Sequenz ist oder die mit der Sequenz hybridisiert, entsprechen.

18. Expressions- oder Klonierungsvektor, der ein Nucleinsäuremolekül gemäß der Definition in Anspruch 16 oder 17 umfaßt.

19. Wirtszelle oder transgener Organismus, der ein Nucleinsäuremolekül gemäß der Definition in Anspruch 16 oder 17 enthält.

20. Verfahren zur Herstellung eines Antigens, eines/einer antigenen Fragments, Komponente oder Vorstufe oder eines/einer funktionell äquivalenten Derivats, Analogons oder Variante davon gemäß der Definition in einem der Ansprüche 1 bis 7, das das Kultivieren einer Wirtszelle, die ein Nucleinsäuremolekül enthält, das für das gesamte Antigen oder einen Teil des Antigens kodiert, unter Bedingungen, unter denen das Antigen exprimiert wird, und die Gewinnung des auf diese Weise gebildeten Antigens umfaßt.

21. Schützendes Metazoen-Parasiten-Antigen oder antigene Fragmente, Komponenten oder Vorstufen davon und funktionell äquivalente Derivate, Analoga oder Varianten davon mit schützender antigener Aktivität gegenüber einem oder mehreren Metazoen-Parasiten, wobei das Antigen dadurch gekennzeichnet ist, daß es Aspartyl-Proteinase-ähnliche und/oder Neutralendopeptidase-ähnliche Aktivitäten aufweist und wobei das Antigen in nativer Form ein integrales Membranprotein oder ein Proteinkomplex ist.

22. Schützendes Metazoen-Parasiten-Antigen oder antigene Fragmente, Komponenten oder Vorstufen davon und funktionell äquivalente Derivate, Analoga oder Varianten davon mit schützender antigener Aktivität gegenüber einem oder mehreren Metazoen-Parasiten, wobei das Antigen ein integrales Membranprotein oder ein Proteinkomplex ist oder einen Teil davon bildet, das/der aus dem Darm von Haemonchus sp erhältlich ist und ein Molekulargewicht von 190 bis 205 kD gemäß einer Bestimmung durch SDS-PAGE auf einem 5 %-igen Gel unter nicht-reduzierenden Bedingungen oder von 35 bis 50 kD gemäß einer Bestimmung durch SDS-PAGE auf einem 10 %-igen Gel unter nicht-reduzierenden Bedingungen aufweist.

## Revendications

1. Antigène protecteur de parasites métazoaires ou des fragments, composants ou précurseurs antigéniques de celui-ci et des dérivés, analogues ou variants de celui-ci fonctionnellement équivalents ayant une activité antigénique protectrice contre un ou plusieurs parasites métazoaires, lequel antigène est caractérisé en ce qu'il est capable de se lier à la pepstatine et sous sa forme d'origine est une protéine membranaire intégrale ou un complexe protéique membranaire intégral.

2. Antigène, fragment, composant ou précurseur antigénique ou dérivé, analogue ou variant de celui-ci fonctionnellement équivalent, selon la revendication 1, caractérisé en outre en ce que:
(a) il possède une activité protéolytique;
(b) sous sa forme d'origine il est placé dans la bordure en brosse de l'intestin du parasite; et
(c) il est capable de se lier à la lectine de germe de blé et aux lectines ayant une spécificité pour la N-acétylgalactosamine à liaison β.

3. Antigène, fragment, composant ou précurseur antigénique ou dérivé, analogue ou variant de celui-ci fonctionnellement équivalent, selon la revendication 1, qui sous sa forme d'origine est capable de se lier à la lectine de Con A, de poix, de lotus, de Helix pomatia, d'arachide et de jacaline.

4. Antigène, fragment, composant, précurseur antigénique ou dérivé, analogue ou variant de celui-ci fonctionnellement équivalent, selon l'une quelconque des revendications 1 à 3 où l'antigène est en outre caractérisé par le fait de posséder une activité semblable à celle de l'aspartyl protéinase et/ou de l'endopeptidase neutre.

5. Antigène, fragment, composant ou précurseur antigénique ou dérivé, analogue ou variant de celui-ci fonctionnellement équivalent, selon l'une quelconque des revendications 1 à 4, pouvant être obtenu des helminthes des espèces Haemonchus, Ostertagia ou Trichostrongylus.

6. Antigène, fragment, composant ou précurseur antigénique ou dérivé, analogue ou variant de celui-ci fonctionnellement équivalent, selon l'une quelconque des revendications 1 à 5 dont la distribution de poids moléculaires établie par électrophorèse sur gel de dodécyl sulfate de sodium et de polyacrylamide est présentée dans le tableau 1, ou un composant de celui-ci.

7. Antigène, fragment, composant ou précurseur antigénique de celui-ci ou dérivé, analogue ou variant de celui-ci fonctionnellement équivalent, selon l'une quelconque des revendications 1 à 6 lequel antigène peut être obtenu de l'intestin de l'espèce Haesmonchus et a un poids moléculaire de 190 à 205 kd déterminé par électrophorèse sur gel de dodécyl sulfate de sodium et de polyacrylamide sur un gel à 5% dans des conditions non réductrices ou de 35 à 50 kd déterminé par électrophorèse sur gel de dodécyl sulfate de sodium et de polyacrylamide sur un gel à 10% dans des conditions non réductrices.

8. Antigène, fragment, composant ou précurseur antigénique ou dérivé, analogue ou variant de celui-ci fonctionnellement équivalent, selon l'une quelconque des revendications 1 à 7, destiné à être utilisé dans la stimulation d'une réponse immune contre des parasites métazoaires chez l'homme ou un animal.

9. Utilisation d'un antigène de parasite métazoaire et des composants, fragments, précurseurs antigéniques et des dérivés, analogues ou variants de celui-ci fonctionnellement équivalents selon l'une quelconque des revendications 1 à 7, pour la préparation d'une composition pour vaccin destinée à être utilisée dans la stimulation d'une réponse immune contre des parasites métazoaires chez l'homme ou un animal.

10. Composition pour vaccin destinée à stimuler une réponse immune contre des parasites métazoaires chez l'homme ou un animal, comprenant un ou plusieurs antigènes ou composants, fragments, précurseurs et des dérivés, analogues ou variants de celui-ci fonctionnellement équivalents selon l'une quelconque des revendications 1 à 7, avec un porteur ou un diluant acceptable du point de vue pharmaceutique.

11. Composition, selon la revendication 10, comprenant un ou plusieurs desdits antigènes avec un ou plusieurs antigènes supplémentaires sélectionnés dans le groupe des antigènes, H110D et H45 et leurs composants.

12. Anticorps, ou fragment de liaison à l'antigène de celui-ci, qui est capable de se lier sélectivement à un antigène, fragment, composant ou précurseur antigénique, ou dérivé, analogue ou variant de celui-ci fonctionnellement équivalent selon l'une quelconque des revendications 1 à 7, ou à l'idiotype d'un dit anticorps se liant à un antigène.

13. Procédé de préparation d'un vaccin destiné à être utilisé dans l'immunisation de l'homme ou d'un animal contre une infection parasitaire métazoaire, ledit procédé comprenant la préparation d'un extrait dudit parasite contenant au moins un antigène protecteur selon l'une quelconque des revendications 1 à 7, de purification dudit antigène à partir de cet extrait en liant ledit antigène à une phase immobilisée incluant un réactif associé de liaison spécifique pour l'antigène et ensuite d'élution dudit antigène de ladite phase immobilisée.

14. Procédé d'identification des antigènes protecteurs chez un parasite métazoaire, comprenant la soumission d'une fraction membranaire intégrale dudit parasite à une chromatographie par affinité en utilisant une phase immobilisée portant une ou plusieurs lectines avec une spécificité pour la N-acétyl-galactosamine.

15. Procédé selon la revendication 13 ou la revendication 14 où ledit réactif associé de liaison spécifique ou lectine est la lectine d'arachide ou de jacaline.

16. Molécule d'acide nucléique comprenant une séquence de nucléotides codant un antigène, fragment, composant ou précurseur antigénique ou un dérivé, analogue ou variant de celui-ci fonctionnellement équivalent selon l'une quelconque des revendications 1 à 7.

17. Molécule d'acide nucléique selon la revendication 16 qui comprend une ou plusieurs séquences de nucléotides correspondant sensiblement à toute la séquence ou une partie de la séquence de nucléotides représentée sur la figure 33 (SEQ ID NO: 1) ou une séquence qui est dégénérée ou sensiblement homologue à ou qui s'hybride avec ladite séquence.

18. Vecteur d'expression ou de clonage comprenant une molécule d'acide nucléique selon la revendication 16 ou la revendication 17.

19. Cellule hôte ou organisme transgénique contenant une molécule d'acide nucléique selon la revendication 16 ou la revendication 17.

20. Procédé de préparation d'un antigène, fragment, composant ou précurseur antigénique ou d'un dérivé, analogue ou variant de celui-ci fonctionnellement équivalent selon l'une quelconque des revendications 1 à 7, qui comprend la culture d'une cellule hôte contenant une molécule d'acide nucléique codant tout ledit antigène ou une partie dudit antigène, dans des conditions dans lesquelles ledit antigène est exprimé et la récupération dudit antigène ainsi produit.

21. Antigène protecteur de parasites métazoaires ou des fragments, composants ou précurseurs antigéniques de celui-ci et des dérivés, analogues ou variants de celui-ci fonctionnellement équivalents ayant une activité antigénique protectrice contre un ou plusieurs parasites métazoaires lequel antigène est caractérisé par le fait de posséder une activité semblable à celle de l'aspartyl protéinase et/ou de l'endopeptidase neutre et sous sa forme d'origine, est une protéine membranaire intégrale ou un complexe protéique membranaire intégral.

22. Antigène protecteur de parasites métazoaires ou des fragments, composants ou précurseurs antigéniques de celui-ci et des dérivés, analogues ou variants de celui-ci fonctionnellement équivalents ayant une activité antigénique protectrice contre un ou plusieurs parasites métazoaires, lequel antigène est une, ou forme une partie d'une, protéine membranaire intégrale ou un complexe protéique membranaire intégral qui peut être obtenu de l'intestin de l'espèce Haemonchus et a un poids moléculaire de 190 à 205 kd déterminé par électrophorèse sur gel de dodécyl sulfate de sodium et de polyacrylamide sur un gel à 5% dans des conditions non réductrices ou de 35 à 50 kd déterminé par électrophorèse sur gel de dodécyl sulfate de sodium et de polyacrylamide sur un gel à 10% dans des conditions non réductrices.
